# EUROPÄISCHE PATENTSCHRIFT

(11) **EP 0 545 511 B1**
(45) Veröffentlichungstag und Bekanntmachung des Hinweises auf die Patenterteilung: **29.05.2002**
(21) Anmeldenummer: 92250351.1
(22) Anmeldetag: 03.12.1992
(51) Int. Cl.: C07D 257/02, C07D 487/18, A61K 49/00, A61K 31/41, C07D 405/06, C07D 405/14

(54) **Verfahren zur Herstellung von mono-N-substituierten Tetraazamakrocyclen**
Process for the preparation of mono-N-substituted tetraazamacrocycles
Procédé pour la préparation de tétraazamacrocycles mono-N-substitués

(30) Priorität: 06.12.1991 DE 4140779
(43) Veröffentlichungstag der Anmeldung: 09.06.1993
(73) Patentinhaber: SCHERING AKTIENGESELLSCHAFT, 13353 Berlin (DE)
(72) Erfinder: Platzek, Johannes, Dr., W-1000 Berlin 33 (DE); Gries, Heinz, Dr., W-1000 Berlin 31 (DE)

(56) Entgegenhaltungen:
- EP-A- 0 232 751
- EP-A- 0 255 471
- EP-A- 0 296 522
- EP-A- 0 389 359
- EP-A- 0 434 345
- EP-A- 0 434 346
- EP-A- 0 448 191
- EP-A- 0 485 045
- HELVETICA CHIMICA ACTA Bd. 69, Nr. 8, 1986, Seiten 2081 - 2086 M. STUDER 'Metal Complexes with Macrocyclic Ligands'

## Beschreibung

Die Erfindung betrifft den in den Patentansprüchen gekennzeichneten Gegenstand, d.h. ein Verfahren zur Herstellung von mono-N-substituierten Tetraazacyclododecan- und -tetradecan-Derivaten.

Mono-N-substituierte Tetraazamakrocyclen der allgemeinen Formel I 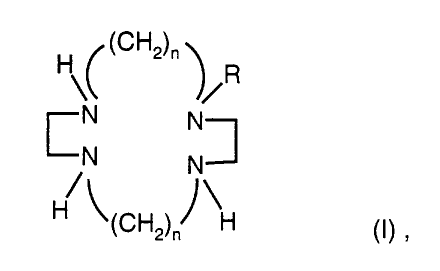 worin
- n: für die Ziffern 2 oder 3,
- R: für eine Gruppe -CR²R³-CHR¹-A, -CHR⁷-CH(OH)-R⁸, -C(X)NHR⁹, -(CH₂)₂-NHSO₂R¹⁰ oder für ein über -CH₂-CH(OH)-K-CH(OH)-CH₂- gebundenes zweites Tetraazacyclododecan- oder -tetradecan-Molekül steht
worin
- R¹: für ein Wasserstoffatom, eine geradkettige oder cyclische C₁-C₆-Alkyl-, eine Phenyl- oder Benzylgruppe - wobei die Phenylgruppe jeweils durch 1 bis 2 Chlor-, Brom-, Nitro-, C₁-C₇-Alkoxy- oder Aralkoxy- oder CO₂R⁴-reste mit R⁴ in der Bedeutung eines Wasserstoffatoms, einer C₁-C₆-Alkyl-, Phenyl- oder Benzylgruppe substituiert sein kann - steht.
- R² und R³: unabhängig voneinander jeweils für R¹ oder eine CO₂R⁴-Gruppe stehen,
- A: für einen CN-, CO₂R⁴- oder 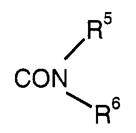 -Rest steht,
worin
- R⁵ und R⁶: unabhängig voneinander jeweils für ein Wasserstoffatom, einen gegebenenfalls durch 1 bis 5 Hydroxygruppe(n) oder 1 bis 2 CO₂R⁴-Reste substituierten gesättigten, ungesättigten, gerad- oder verzweigtkettigen oder cyclischen, gegebenenfalls durch 1 bis 8 Sauerstoffatom(e) oder 1 bis 3 Phenylen- oder Phenylenoxygruppe(n) unterbrochenen Kohlenwasserstoffrest mit bis zu 16 C-Atomen, für gegebenenfalls durch 1 bis 3 Hydroxy- oder C₁-C₆-Alkoxygruppen substituierte Phenyl- oder Benzylreste oder R⁵ und R⁶ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3, gegebenenfalls durch 1 bis 3 Hydroxyreste substituierte C₁-C₆-Alkylreste, wobei gegebenenfalls vorhandene Hydroxy- und/oder Carboxylgruppen gegebenenfalls geschützt sind,
- R⁷ und R⁸: unabhängig voneinander jeweils für ein Wasserstoffatom, einen gegebenenfalls durch 1 bis 10 Sauerstoffatom(e), eine Phenylen-, Phenylenoxy- oder Phenylendioxygruppe unterbrochenen C₁-C₂₀-Alkylrest, der gegebenenfalls durch 1 bis 3 C₁-C₆-Alkyl-, 1 bis 3 Trifluormethyl, 1 bis 7 Hydroxy-, 1 bis 3 C₁-C₇-Alkoxy- oder -Aralkoxy-, 1 bis 2 CO₂R⁴- und/oder 1 bis 2, gegebenenfalls durch 1 bis 2 Chlor-, Brom-, Nitro- oder C₁-C₆-Alkoxyreste substituierte Phenoxy- oder Phenylgruppen substituiert ist, stehen, wobei die gegebenenfalls vorhandenen Hydroxyreste gegebenenfalls in geschützter Form vorliegen,
- X: für ein Sauerstoff- oder Schwefelatom und
- R⁹: für eine Phenyl-, 1- oder 2-Naphthyl- oder geradkettige oder cyclische C₁-C₆-Alkylgruppe,
- R¹⁰: für eine C₁-C₆-Alkyl-, -CF₃- oder eine gegebenenfalls durch einen C₁-C₆-Alkyl-, Chlor-, Brom- oder Nitrorest substituierte Phenylgruppe, und
- K: für eine gegebenenfalls durch 1 bis 6 Sauerstoffatome, 1 bis 2 Phenylen-,
Phenylenoxy- oder Phenylendioxygruppen unterbrochene, gegebenenfalls durch 1 bis 6 Hydroxy-, Hydroxyalkyl- oder 1 bis 6 C₁-C₇-Alkoxy-, Benzyloxy- oder Aralkoxygruppen substituierte C₀-C₁₆-Alkylenkette stehen, wobei Carboxyl- und Hydroxygruppen gegebenenfalls in geschützter Form vorliegen, sind wichtige Vorstufen von Tri-N-carboxyalkyl-, bevorzugt Tri-N-carboxymethyl-, substituierten Tetraazamakrocyclen, die in Form ihrer Komplexe mit den Metallionen der Ordnungszahlen 21 bis 29, 31, 32, 38, 39, 42-44, 49 oder 57-83 Verwendung als Diagnostika und Therapeutika finden (siehe Europäische Patentanmeldung Publikations-Nr. 255471).

Wegen ihrer Bedeutung als Schlüsselverbindungen für diese Komplexe, vor allem für die bevorzugten NMR-Diagnostika (Macrocyclic Chemistry Congress, Hamburg 1988), ist ihre Herstellung auf unterschiedliche Art und Weise versucht worden, ohne daß bisher ein befriedigender Syntheseweg gefunden werden konnte.

So ist z.B. eine statistische Mono-alkylierung- bzw. -acylierung von unsubstituierten Tetraazamakrocyclen beschrieben worden, die jedoch wegen des zu verwendenden großen Überschusses an relativ kostbarem Ausgangsamin, zum Teil sehr aufwendiger chromatographischer Abtrennung des Produkts vom Ausgangsmaterial sowie meist recht mäßiger Ausbeuten zumindest für die Herstellung größerer Substanzmengen nicht geeignet ist [siehe Kaden, Helv. Chim. Acta 69, 2081 (1986); Kimura, J. Chem. Soc. Chem. Commun. 1158 (1986); Kaden, Top. Curr. Chem. 121, 157 (1984); Europäische Patentanmeldungen Nr. 296522 und Nr. 3553450].

Will man - im Gegensatz zur oben beschriebenen statistischen - eine gezielte Monosubstitution durchführen, so sind zwei Varianten möglich:
a) Umsetzung eines mit drei Stickstoff-Schutzgruppen versehenen Tetraazamakrocyclus, der durch statistische Trisubstitution erhalten wurde,
b) Umsetzung eines mit drei Stickstoff-Schutzgruppen versehenen Tetraazamakrocyclus, der durch gezielte Synthese hergestellt wurde.

Bei der erstgenannten Variante wird die Schutzgruppen (z.B. Tosylat, Benzoat) an drei Stickstoffatomen tragende Vorstufe durch statistische Trisubstitution eines unsubstituierten Tetraazamakrocyclus hergestellt, so daß die oben genannten Nachteile einer statistischen Umsetzung wie geringe Ausbeuten, Trennprobleme (vor allem bei der Herstellung größerer Substanzmengen) auch hier auftreten [siehe z.B. Macrocyclic Chemistry Congress, Hamburg 1988]. Nach der sich anschließenden gezielten Monosubstitution zur Einführung des Substituenten R [Ciampolini, J. Chem. Soc. Chem. Commun. 998 (1984): Kaden, Helv. Chim. Acta 66, 861 (1983); Basefield, Inorg. Chem. 25, 4663 (1986)] müssen die Schutzgruppen an den drei Stickstoffatomen, z.B. mittels Alkalimetall in Ammoniak [Helv. Chim. acta, 56, 2216 (1973); Helv. Chim. acta 59, 1566 (1976); J. Org. Chem. 53, 3521 (1988)], Lithiumaluminiumhydrid (F. Vögtle; Liebigs Ann. Chem. (1977), 1344], Red-Al^{(R)} [E.H. Gold, J. Org. Chem. (1972), 37. 2208], Na-Hg [M. Kellog, J. Org. Chem. 1984, 49, 110], Elektrolyse [M. Hesse, Helv. Chim. Acta 71 (1988), 7, 1708] oder Bromwasserstoffsäure/Phenol/Eisessig [N.G. Lukyanenko, Synthesis, 1988, 355] entfernt werden. Diese Verfahren der Schutzgruppenabspaltung sind im allgemeinen mit schlechten Ausbeuten verbunden, limitieren die Ansatzgröße hinsichtlich der einzusetzenden Reagenzmenge (z.B. bei der Na-Hg-Methode) und sind vor allem bei Substituenten, die empfindliche Gruppen (z.B. Hydroxyalkyl) tragen, nicht anwendbar.

Verfährt man nach Variante b), das heißt will man die Schutzgruppen an drei Stickstoffatomen tragende Tetraazamakrocyclus-Vorstufe durch gezielte Synthese herstellen, so geht man von zwei Reaktanten aus, die nach literaturbekannten Methoden [z.B. Richman, Org. Synthesis 58, 86 (1978); Atkins, J. Amer. Chem. Soc. 96, 2268 (1974)] cyclisiert werden: einer der beiden Reaktanten enthält ein geschütztes Stickstoffatom und trägt am Kettenende zwei Fluchtgruppen (z.B. Brom-, Mesyloxy-, Tosyloxy-, Triflat- oder Alkoxycarbonylgruppen), die von den endständigen Stickstoffatomen des zweiten Reaktanten, einer - anders als der erste Reaktant - geschützten Triazaverbindung, nukleophil verdrängt werden.

[Wird ein Reaktant mit zwei endständigen Estergruppen verwendet, so müssen die durch die Cyclisierung entstandenen zwei Amidgruppierungen - vorzugsweise mit Diboran in THF - reduziert werden. Speziell diese Cyclisierungsvariante ist jedoch für die Herstellung größerer Substanzmengen ungeeignet, da diese Umsetzung in möglichst hoher Verdünnung durchzuführen ist, um z.B. Polymerisationsreaktionen zu vermeiden: siehe Tabushi, Tetrahed. Lett. 12, 1049 (1977); Kaden, Inorg. Chem. 25, 321 (1986). Auch die Aufarbeitung der folgenden Diboran-Reduktion ist - wieder vor allem bei größeren Ansätzen - nicht unproblematisch.]

Nach Abspaltung der einen Schutzgruppe kann die so freigesetzte Iminogruppierung alkyliert bzw. acyliert werden. Als Beispiel sei die Umsetzung des Dinatriumsalzes des N, N', N"-Tris-(p-tolylsulfonyl)diethylentriamins [Ciampolini, J.Chem. Soc Chem. Commun. 998 (1984)] mit N-Bis-(2-methan-sulfonyloxy-ethyl)-triphenylmethylamin in Dimethylformamid bei 80 - 150 °C mit nachfolgender Abspaltung der Tritylgruppe unter sauren Bedingungen genannt. Die Ausbeuten der beiden Reaktionsstufen sind im allgemeinen schlecht. Auch diese Variante b) ist mit den unter a) genannten Nachteilen für die Abspaltung der drei aus dem zweiten Reaktanten stammenden Schutzgruppe behaftet.

Neben den bisher vorgestellten Verfahren der statistischen und der gezielten Monosubstitution ist auch eine gezielte Ringsynthese, bei der der gewünschte Substituent R schon in einem der beiden in die Cyclisierungsreaktion einzusetzenden Reaktanten enthalten ist, möglich. (Siehe z.B. EP-A-0232751)

Neben der bereits weiter oben geschilderten Problematik der Schutzgruppenabspaltung hat es sich gezeigt, daß die so durchgeführten Cyclisierungen im allgemeinen mit geringeren Ausbeuten - verglichen mit den Umsetzungen der nur mit Schutzgruppen versehenen Reaktionspartner - verlaufen [siehe Atkins, J. Amer. Chem. Soc. 96, 2258 (1974); Richman, Org. Synthesis 58, 86 (1978); Fabbrizzi, Inorg. Chem. 25, 4131 (1986); Gazetta, Chimica Italiana 115, 399 (1985)]. Außerdem müssen die den Substituenten R tragenden Reaktanten erst in einer oft mehrere Stufen umfassenden Reaktionsfolge speziell synthetisiert werden [siehe z.B. Bulkowski, J. Org. Chem. 47, 412 (1982)].

Die in EP-A-0434345, EP-A-0434346, EP-A-0448191 und EP-A-0485045 beschriebenen Verfahren zur Herstellung von tri-N-carboxymethyl- substituierten Tetraazacyclododecanen offenbaren die Umsetzung eines tri-N-substituierten Makrocyclus, ein Mono-N-substituierter Tetraazamakrocyclus der allgemeinen Formel I wird nicht verwendet.

EP-A-0489359 beschreibt die Synthese monofunktionalisierter cyclischer Tetraamine, ausgehend von physiologisch nicht unbedenklichen tri-N-geschützten B-, P-, Cr-, Mooder W-Komplexen.

Trotz vielfältiger Bemühungen ist es daher bisher nicht gelungen, einen befriedigenden Syntheseweg für Mono-N-substituierte Tetraazamakrocyclen der allgemeinen Formel I, die als Schlüsselverbindungen für die als wertvolle NMR- und Röntgen-Kontrastmittel dienenden Tri-N-carboxyalkyl-Metallkomplexe anzusehen sind, zu finden.

Der Erfindung liegt somit die Aufgabe zugrunde, ein derartiges Verfahren, das vor allem für die Herstellung größerer Substanzmengen geeignet ist, zur Verfügung zu stellen.

Diese Aufgabe wird durch die vorliegende Erfindung gelöst.

Es wurde gefunden, daß man überraschenderweise eine selektive Monofunktionalisierung zu den mono-N-substituierten Tetraazamakrocyclen der allgemeinen Formel I 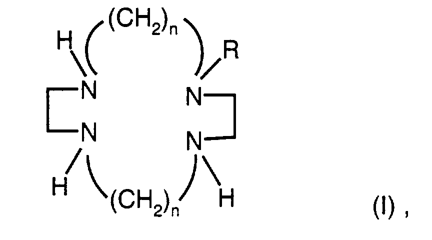 worin
- n: für die Ziffern 2 oder 3,
- R: für eine Gruppe -CR²R³-CHR¹-A, -CHR⁷-CH(OH)-R⁸, -C(X)NHR⁹, -(CH₂)₂-NHSO₂R¹⁰ oder für ein über -CH₂-CH(OH)-K-CH(OH)-CH₂- gebundenes zweites Tetraazacyclododecan- oder -tetradecan-Molekül steht
worin
- R¹: für ein Wasserstoffatom, eine geradkettige oder cyclische C₁-C₆-Alkyl-, eine Phenyl- oder Benzylgruppe - wobei die Phenylgruppe jeweils durch 1 bis 2 Chlor-, Brom-, Nitro-, C₁-C₇-Alkoxy- oder Aralkoxy- oder CO₂R⁴-reste mit R⁴ in der Bedeutung eines Wasserstoffatoms, einer C₁-C₆-Alkyl-, Phenyl- oder Benzylgruppe substituiert sein kann - steht
- R² und R³: unabhängig voneinander jeweils für R¹ oder eine CO₂R⁴-Gruppe stehen,
- A: für einen CN-, CO₂R⁴- oder 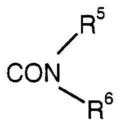 -Rest steht,
worin
R⁵ und R⁶ unabhängig voneinander jeweils für ein Wasserstoffatom, einen gegebenenfalls durch 1 bis 5 Hydroxygruppe(n) oder 1 bis 2 CO₂R⁴-Reste substituierten gesättigten, ungesättigten, gerad- oder verzweigtkettigen oder cyclischen, gegebenenfalls durch 1 bis 8 Sauerstoffatom(e) oder 1 bis 3 Phenylen- oder Phenylenoxygruppe(n) unterbrochenen Kohlenwasserstoffrest mit bis zu 16 C-Atomen, für gegebenenfalls durch 1 bis 3 Hydroxy- oder C₁-C₆-Alkoxygruppen substituierte Phenyl- oder Benzylreste oder R⁵ und R⁶ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3, gegebenenfalls durch 1 bis 3 Hydroxyreste substituierte C₁-C₆-Alkylreste, wobei gegebenenfalls vorhandene Hydroxy- und/oder Carboxylgruppen gegebenenfalls geschützt sind,
R⁷ und R⁸ unabhängig voneinander jeweils für ein Wasserstoffatom, einen gegebenenfalls durch 1 bis 10 Sauerstoffatom(e), eine Phenylen-, Phenylenoxy- oder Phenylendioxygruppe unterbrochenen C₁-C₂₀-Alkylrest, der gegebenenfalls durch 1 bis 3 C₁-C₆-Alkyl-, 1 bis 3 Trifluormethyl, 1 bis 7 Hydroxy-, 1 bis 3 C₁-C₇-Alkoxy- oder -Aralkoxy-, 1 bis 2 CO₂R⁴- und/oder 1 bis 2, gegebenenfalls durch 1 bis 2 Chlor-, Brom-, Nitro- oder C₁-C₆-Alkoxyreste substituierte Phenoxy- oder Phenylgruppen substituiert ist, stehen, wobei die gegebenenfalls vorhandenen Hydroxyreste gegebenenfalls in geschützter Form vorliegen,
- X: für ein Sauerstoff- oder Schwefelatom und
- R⁹: für eine Phenyl-, 1- oder 2-Naphthyl- oder geradkettige oder cyclische C₁-C₆-Alkylgruppe,
- R¹⁰: für eine C₁-C₆-Alkyl-, -CF₃- oder eine gegebenenfalls durch einen C₁-C₆-Alkyl-, Chlor-, Brom- oder Nitrorest substituierte Phenylgruppe, und
- K: für eine gegebenenfalls durch 1 bis 6 Sauerstoffatome, 1 bis 2 Phenylen-,
Phenylenoxy- oder Phenylendioxygruppen unterbrochene, gegebenenfalls durch 1 bis 6 Hydroxy-, Hydroxyalkyl- oder 1 bis 6 C₁-C₇-Alkoxy-, Benzyloxy- oder Aralkoxygruppen substituierte C₀-C₁₆-Alkylenkette stehen, wobei Carboxyl- und Hydroxygruppen gegebenenfalls in geschützter Form vorliegen,
erzielt, wenn man Tetraazatricyclotridecan- bzw. -pentadecan der allgemeinen Formel II 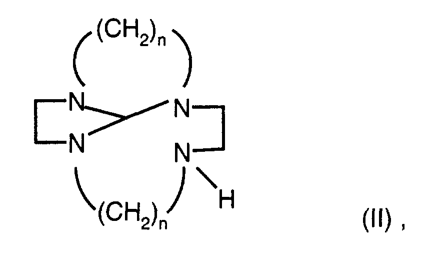 mit einem entsprechenden Edukt der allgemeinen Formel 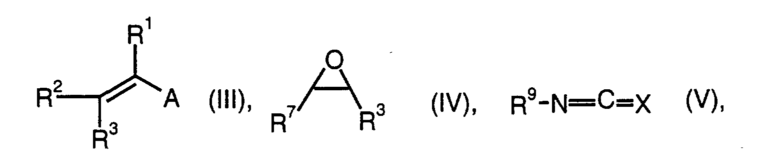 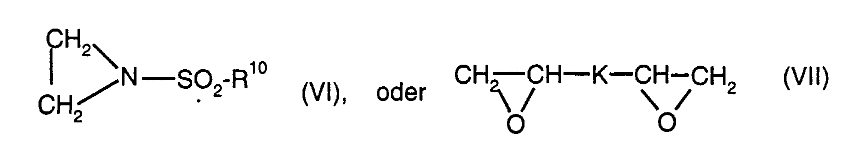 worin
R¹-R¹⁰, A, X und K die oben angegebene Bedeutung haben,
mit oder ohne Lösungsmittel bei 0 bis 220 °C, innerhalb von 1 bis 48 Stunden, gegebenenfalls bei einem Druck bis zu 100 atm. umsetzt, anschließend das so erhaltene Reaktionsgemisch nach Abkühlung auf -20 °C bis 80 °C, mit einem Gemisch Wasser/organisches Lösungsmittel, ausgewählt aus Methanol, Ethanol, iso-Propanol, Tetrahydrofuran oder Dioxan, versetzt und 0,5 bis 12 Stunden, bei -20 °C bis Raumtempratur rührt, dann die so gebildeten - gewünschtenfalls zu isolierenden - eine Formylgruppe an einem Stickstoffatom tragenden Zwischenprodukte durch Zugabe einer anorganischen Base, ausgewählt aus Lithium-, Natrium-, Kalium-, Barium- oder Calciumhydroxid oder einer Mineralsäure, ausgewählt aus Salz- Schwefel- oder Bromwasserstoffsäure bei 0 bis 150 °C, innerhalb von 1 bis 72, unter Rühren- gegebenenfalls gefolgt von anschließender Schutzgruppenentfernung in an sich üblicher Weise - zum Endprodukt der Formel I umsetzt und anschließend in an sich bekannter Weise isoliert.

Die als Intermediate verwendeten Tetraazatricyclotridecane- bzw. -pentadecane der allgemeinen Formel II sind nach literaturbekannten Methoden zugänglich, z.B. indem man 1,4,7,10-Tetraazatricyclododecan- bzw. 1,4,8,11-Tetraazacyclotetradecan mit Dimethylformamid-dimethylacetal umsetzt (US-Patentschriften 4,085,106 und 4,130,715), J. Am. Chem. Soc. 102, 6364 (1980), EP 292 689.

Vorteilhafterweise bezieht man diesen Reaktionsschritt in das erfindungsgemäße Verfahren ein, ohne daß die Intermediate der allgemeinen Formel II isoliert werden müssen ("Eintopfreaktion").

Eine spezielle Ausführungsform des erfindungsgemäßen Verfahrens ist die Herstellung von Verbindungen der allgemeinen Formel I mit
R in der Bedeutung einer 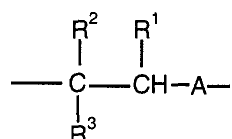 Gruppe,
worin R¹, R²,R³ und A die obengenannte Bedeutung haben, dadurch gekennzeichnet, daß man Tetraazatricyclotridecan bzw. -pentadecan mit einem Edukt der allgemeinen Formel III 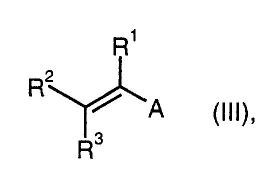 worin R¹, R²,R³ und A die oben angegebene Bedeutung haben, wobei gegebenenfalls vorhandene Hydroxy- und/oder Carboxylgruppen gegebenenfalls geschützt sind, mit oder ohne Lösungsmittel, wobei als Lösungsmitttel bevorzugt aprotische Lösungsmittel wie z.B. Benzol, Toluol, Dichlormethan, Tetrahydrofuran, Dioxan, Acetonitril, Dimethylformamid, Hexan oder Ether verwendet erden, bei 0 °C bis 210 °C, vorzugsweise 50 °C bis 180 °C (wobei im Falle der höheren Umsetzungstemperatur das gegebenenfals zum Lösen des zugesetzten Edukts der allgemeinen Formel III verwendete Lösungsmittel vorher im Vakuum abdestilliert worden ist), innerhalb von 12 bis 48, vorzugsweise 5 bis 12 Stunden, umsetzt, anschließend das so erhaltene Reaktionsgemisch auf -20 °C bis 80 ° C, vorzugsweise 0 ° C bis 30 ° C, abkühlt, mit einem Gemisch Wasser/organisches Lösungsmittel wie z.B. Methanol, Ethanol, iso-Propanol, Tetrahydrofuran oder Dioxan versetzt und 0,5 bis 12 Stunden, vorzugsweise 0,5 bis 3 Stunden, bei -20 °C bis Raumtemperatur, vorzugsweise 0 °C bis Raumtemperatur, rührt, dann das so gebildete - gewünschtenfalls zu isolierende - eine Formylgruppe an einem Stickstoffatom tragende Zwischenprodukt durch Zugabe einer anorganischen Base wie z.B. Lithium-, Natrium-, Kalium-, Barium- oder Calciumhydroxid, bevorzugt Natrium- und Kaliumhydroxid, oder einer Mineralsäure wie z.B. Salz-, Schwefel- oder Bromwasserstoffsäure, bevorzugt Salzsäure, bei 0 °C bis 150 °C, vorzugsweise Raumtemperatur bis 120 °C, innerhalb von 1 bis 72 Stunden, vorzugsweise 6 bis 24 Stunden, unter Rühren - gegebenenfalls gefolgt von anschließender Schutzgruppenentfernung in an sich üblicher Weise - zum Endprodukt der Formel I umsetzt und anschließend in an sich bekannter Weise, vorzugsweise als Hydrochlorid, isoliert.

Steht R¹ für eine C₁-C₆-Alkylgruppe, so ist die Methyl- und Ethylgruppe bevorzugt. Weitere bevorzugte Reste R¹ sind das Wasserstoffatom und der gegebenenfalls substituierte Phenylrest; als bevorzugte Substituenten am Phenylring sind die Nitrogruppe, der C₁-C₇-Alkoxyrest, vor allem der Methoxyund Ethoxyrest, und der CO₂R⁴-Rest, wobei R⁴ bevorzugt für ein Wasserstoffatom, eine Methyl-, Ethyl-, t-Butyl- oder Benzylgruppe steht, zu nennen.

Als bevorzugte für R⁵ und R⁶ stehende Reste seien das Wasserstoffatom, die Methyl-, Ethyl-, 2-Hydroxyethyl-, 2-Hydroxy-1-(hydroxymethyl)-ethyl-. 1-(Hydroxymethyl)-ethyl-, )-ethyl-, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-, 3- und 4-Hydroxy-2-methylbutyl-, 2- und 3- Hydroxyisobutyl-, 2,3,4-Trihydroxybutyl-, 1,2,4-Trihydroxybutyl-, Pentyl-, Cyclopentyl-, 2-Methoxyethyl-, Hexyl-, Decyl-, Tetradecyl-, Triethylenglykolmethylether-, Tetraethylenglykolmethylether- und Methoxybenzylgruppe genannt. Der Amidrest kann auch ein unter Einschluß des Amid-Stickstoffs gebildeter heterocyclischer 5- oder 6-Ring sein. Beispielhaft genannt seien: der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Im Substrat III gegebenenfalls vorhandene Carboxyl- und/oder Hydroxygruppen liegen vorzugsweise in geschützter Form vor.

Als Säureschutzgruppe kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl-, Diphenyl-t-butylsilylgruppe infrage.

Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle von Polyolen können die Hydroxygruppen auch in Form von Ketalen mit z.B. Aceton, Acetaldehyd, Cyclohexanon oder Benzaldehyd geschützt sein.

Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Eine weitere spezielle Ausführungsform des erfindungsgemäßen Verfahrens ist die Herstellung von Verbindungen der allgemeinen Formel I mit R in der Bedeutung einer
worin 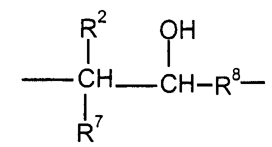 Gruppe,
R⁷ und R⁸ die oben genannte Bedeutung haben, dadurch gekennzeichnet, daß man Tetraazatricyclotridecan bzw. -pentadecan mit einerm Edukt der allgemeinen Formel IV 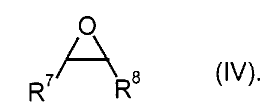 worin
R⁷ und R⁸ die oben angegebene Bedeutung, wobei gegebenenfalls vorhandene Hydroxy- und/oder Carboxylgruppen gegebenenfalls geschützt sind, mit oder ohne Lösungsmittel, wobei als Lösungsmittel bevorzugt aprotische Lösungsmittel wie z. B. Benzol, Toluol, Dichlormethan, Tetrahydrofuran, Dioxan, Acetonitril, Dimethylformamid, Dimethylacetamid, Dimethylsulfoxid, Hexan oder Ether verwendet werden, bei 0 °C bis 220 °C, vorzugsweise 50 °C bis 180 °C (wobei im Falle der höheren Umsetzungstemperatur das gegebenenfalls zum Lösen des zugesetzten Edukts der allgemeinen Formel IV verwendete Lösungsmittel
vorher im Vakuum abdestilliert worden ist) oder in einem Autoklaven bei einem Überdruck von bis 100 atm. innerhalb von 1 bis 48 Stunden, vorzugsweise 5 bis 12 Stunden, umsetzt, anschließend das so erhaltene Reaktionsgemisch auf -20 °C bis 80 °C, vorzugsweise 0 °C bis 30 °C, abkühlt, mit einem Gemisch Wasser/organisches Lösungsmittel wie z.B. Methanol, Ethanol, iso-Propanol, Tetrahydrofuran oder Dioxan versetzt und 0,5 bis 12 Stunden, vorzugsweise 0,5 bis 3 Stunden, bei -20 °C bis Raumtemperatur, vorzugsweise 0 °C bis Raumtemperatur, rührt, dann die so gebildeten - gewünschtenfalls zu isolierende - eine Formylgruppe an einem Stickstoffatom tragenden Zwischenprodukte durch Zugabe einer anorganischen Base wie z.B. Lithium-, Natrium-, Kalium-, Barium- oder Calciumhydroxid, bevorzugt Natrium- und Kaliumhydroxid, oder einer Mineralsäure wie z.B. Salz-, Schwefel- oder Bromwasserstoffsäure, bevorzugt Salzsäure, bei 0 °C bis 150 °C, vorzugsweise Raumtemperatur bis 120 °C, innerhalb von 1 bis 72 Stunden, vorzugsweise 6 bis 24 Stunden, unter Rühren - gegebenenfalls gefolgt von anschließender Schutzgruppenentfernung in an sich üblicher Weise - zum Endprodukt der Formel I umsetzt und anschließend in an sich bekannter Weise, vorzugsweise als Hydrochlorid, isoliert.

Bevorzugte Reste R⁷ und R⁸ sind das Wasserstoffatom, die Methyl-, Ethyl-, 2-Hydroxyethyl-, Hydroxymethyl, 2-Hydroxy-1-(hydroxymethyl)-ethyl-, 1-(Hydroxymethyl)ethyl-, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- 2-Methoxyethyl-, Hexyl-, Decyl-, Tetradecyl-, Triethylenglykolmethylether-, Tetraethylenglykolmethylether- und Methoxybenzylgruppe sowie die
-CH₂-O-C₁₁H₂₂-OH-,
-CH₂-O-C₆H₄-O-(CH₂CH₂O)₂-CH₃-,
-CH₂-O-C₆H₄-O-(CH₂CH₂O)₃-C₅H₁₁-,
-CH₂-O-C₆H₄-O-C₄H₈-OH-,
-(CH₂CH₂O)₅-CH₃-,
-C₉H₁₈-OH-,
-C₉H₁₈-COOH-,
-CH₂-O-C₆H₄-O-C₆H₁₂-COOH-,
-CH₂-O-C₆H₄-O-C₄H₈-O-CH₂-CHOH-CH₂OH-,
-(CH₂CH₂-O)₃-C₅H₁₁-,
-CH₂-O-C₁₀H₂₀-COOH-,
-CH₂-O-C₆H₄-Cl-,
-CH₂-O-C₆H₄NO₂-,
-CH₂-O-C₆H₃Cl₂-,
-CH₂-O-C₆H₄-COOH-,
-CH₂-O-C₆H₄-O-CH₂-COOH-,
-CH₂-O-C₆H₄-C₅H₁₁-,
-CH₂-OCH₂-CHOH-CH₂OH und
-CHOH-CH₂OH-Gruppe.

Bei der Verwendung von flüchtigen Epoxiden wie z.B. Ethylen- oder Propylenoxid wird die Reaktion im Autoklaven durchgeführt.

Im Substrat IV gegebenenfalls vorhandene Carboxyl- und/oder Hydroxygruppen liegen vorzugsweise in geschützter Form, wie oben im Falle des Substrats III beschrieben, vor.

Eine weitere spezielle Ausführungsform des erfindungsgemäßen Verfahrens ist die Herstellung von Verbindungen der allgemeinen Formel I mit R in der Bedeutung eines 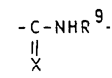 Restes,
worin
X und R⁹ die oben genannte Bedeutung haben, dadurch gekennzeichnet, daß man Tetraazatricyclotridecan bzw. -pentadecan mt einem Edukt der allgemeinen Formel V

R⁹-N=C=X

worin
X und R⁹ die oben angegebene Bedeutung haben,
mit oder ohne Lösungsmittel, wobei als Lösungsmittel bevorzugt aprotische Lösungsmittel wie z.B. Benzol, Toluol, Dichlormethan, Tetrahydrofuran, Dioxan, Acetonitril, Dimethylformamid, Hexan oder Ether verwendet werden, bei 0 °C bis 180 °C, vorzugsweise Raumtemperatur bis 150 °C (wobei im Falle der höheren Umsetzungstemperatur das gegebenenfalls zum Lösen des zugesetzten Edukts der allgemeinen Formel V verwendete Lösungsmittel vorher im Vakuum abdestilliert worden ist), innerhalb von 1 bis 48 Stunden, vorzugsweise 5 bis 12 Stunden, umsetzt, anschließend das so erhaltene Reaktionsgemisch auf -20 °C bis 80 °C, vorzugsweise 0 °C bis 30 °C. abkühlt, mit einem Gemisch Wasser/organisches Lösungsmittel wie z.B. Methanol, Ethanol, iso-Propanol, Tetrahydrofuran oder Dioxan versetzt und 0,5 bis 12 Stunden, vorzugsweise 0,5 bis 3 Stunden, bei -20 °C bis Raumtemperatur, vorzugsweise 0 °C bis Raumtemperatur, rührt, dann das so gebildete - gewünschtenfalls zu isolierende - eine Formylgruppe an einem Stickstoffatom tragende Zwischenprodukt durch Zugabe einer anorganischen Base wie z.B. Lithium-, Natrium-. Kalium-, Barium- oder Calciumhydroxid, bevorzugt Natrium- und Kaliumhydroxid, oder einer Mineralsäure wie z.B. Salz-, Schwefel- oder Bromwasserstoffsäure, bevorzugt Salzsäure, bei 0 °C bis 150 °C, vorzugsweise Raumtemperatur bis 120 °C, innerhalb von 1 bis 72 Stunden, vorzugsweise 6 bis bis 24 Stunden, unter Rühren zum Endprodukt der Formel I umsetzt und anschließend in an sich bekannter Weise, vorzugsweise als Hydrochlorid, isoliert.

Noch eine weitere spezielle Ausführungsform des erfindungsgemäßen Verfahrens ist die Herstellung von Verbindungen der allgemeinen Formel I mit R in der Bedeutung eines -(CH₂)₂ -NH-SO₂-R¹⁰ Restes,
worin
R¹⁰ die oben genannte Bedeutung hat, dadurch gekennzeichnet, daß man Tetraazatricyclotridecan bzw. -pentadecan mit einem Edukt der allgemeinen Formel VI 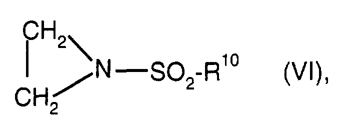 worin
R¹⁰ die oben angegebene Bedeutung hat mit Lösungsmittel, wobei als Lösungsmittel bevorzugt aprotische Lösungsmittel wie z.B. Benzol. Toluol, Dichlormethan, Tetrahydrofuran, Dioxan, Acetonitril, Dimethylformamid, Hexan oder Ether verwendet werden, bei 0 °C bis 180 °C, vorzugsweise Raumtemperatur bis 150 °C, (wobei im Falle der höheren Umsetzungstemperatur das verwendete Lösungsmittel vorher im Vakuum abdestilliert worden ist), innerhalb von 1 bis 48 Stunden, vorzugsweise 5 bis 12 Stunden, umsetzt, anschließend das so erhaltene Reaktionsgemisch auf -20 °C bis 80 °C, vorzugsweise 0 °C bis 30 °C, abkühlt, mit einem Gemisch Wasser/organisches Lösungsmittel wie z.B. Methanol, Ethanol, iso-Propanol, Tetrahydrofuran oder Dioxan versetzt und 0,5 bis 12 Stunden, vorzugsweise 0,5 bis 3 Stunden, bei -20 °C bis Raumtemperatur, vorzugsweise 0 °C bis Raumtemperatur, rührt, dann das so gebildete - gewünschtenfalls zu isolierende - eine Formylgruppe an einem Stickstoffatom tragende Zwischenprodukt durch Zugabe einer anorganischen Base wie z.B. Lithium-, Natrium-, Kalium-, Barium- oder Calciumhydroxid, bevorzugt Natrium- und Kaliumhydroxid, oder einer Mineralsäure wie z.B. Salz-, Schwefel- oder Bromwasserstoffsäure, bevorzugt Salzsäure, bei 0 °C bis 150 °C, vorzugsweise Raumtemperatur bis 120 °C, innerhalb von 1 bis 72 Stunden, vorzugsweise 6 bis 24 Stunden, unter Rühren zum Endprodukt der Formel I umsetzt und anschließend in an sich bekannter Weise, vorzugsweise als Hydrochlorid, isoliert.

Bevorzugte Reste R¹⁰ sind der Phenyl- und 4-Methylphenylrest.

Eine weitere spezielle Ausführungsform des erfindungsgernäßen Verfahrens ist die Herstellung von Dimeren, das heißt von Verbindungen der allgemeinen Formel I mit R in der Bedeutung eines über eine
Bis(β-hydroxy)-alkylenkette 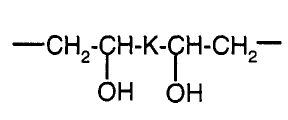 gebundenen zweiten 1,4,7,10-Tetraazacyclododecan-oder -oder -tetradecan-Moleküls,
worin
K die oben genannte Bedeutung hat, dadurch gekennzeichnet, daß man Tetraazatricyclotridecan bzw. -pentadecan mit einem Edukt der allgemeinen Formel VII 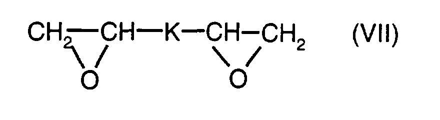 worin
K die oben angegebene Bedeutung hat, wobei gegebenenfalls vorhandene Hydroxygruppen gegebenenfalls geschützt sind, mit oder ohne Lösungsmittel, wobei als Lösungsmittel bevorzugt aprotische Lösungsmittel wie z.B. Benzol, Toluol, Dichlormethan, Tetrahydrofuran, Dioxan, Acetonitril, Dimethylformamid, Hexan oder Ether verwendet werden, bei 0 °C bis 220 °C, vorzugsweise 50 °C bis 180 °C, (wobei im Falle der höheren Umsetzungstemperatur das gegebenenfalls zum Lösen des zugesetzten Edukts der allgemeinen Formel VII verwendete Lösungsmittel vorher im Vakuum abdestilliert worden ist) oder in einem Autoklaven bei einem Überdruck von 1 bis 100 atm. innerhalb von 1 bis 48 Stunden, vorzugsweise 5 bis 12 Stunden, umsetzt, anschließend das so erhaltene Reaktionsgemisch auf -20 °C bis 80 °C, vorzugsweise 0 °C bis 30 °C, abkühlt, mit einem Gemisch Wasser/organisches Lösungsmittel, wie z.B. Methanol, Ethanol, iso-Propanol, Tetrahydrofuran oder Dioxan versetzt und 0,5 bis 12 Stunden, vorzugsweise 0,5 bis 3 Stunden, bei -20 °C bis Raumtemperatur, vorzugsweise 0 °C bis Raumtemperatur, rührt, dann das so gebildete - gewünschtenfalls zu isolierende - eine Formylgruppe an einem Stickstoffatom tragende Zwischenprodukt durch Zugabe einer anorganischen Base wie z.B. Lithium-, Natrium-, Kalium-, Barium- oder Calciumhydroxid, bevorzugt Natrium- und Kaliumhydroxid, oder einer Mineralsäure wie z.B. Salz-, Schwefel-oder Bromwasserstoffsäure, bevorzugt Salzsäure, bei 0 °C bis 150 °C, vorzugsweise Raumtemperatur bis 120 °C. innerhalb von 1 bis 72 Stunden, vorzugsweise 6 bis 24 Stunden, unter Rühren - gegebenenfalls gefolgt von anschließender Schutzgruppenentfernung in an sich üblicher Weise - zum Endprodukt der Formel I umsetzt und anschließend in an sich bekannter Weise, vorzugsweise als Hydrochlorid, isoliert.

Bevorzugte Brückenglieder K sind beispielsweise
-C₂H₄-
-CH₂-
-CH₂-O-CH₂-
-CH₂-O-CH₂-CH₂-O-CH₂-
-CH₂-O-(CH₂CH₂O)₂-CH₂-
-CHOH-
-CHOH-CHOH-
-CHOH-CHOH-CHOH-
-CH₂-O-CH₂-CHOH-CH₂-O-CH₂-
-CH₂-O-C₆H₄-O-CH₂-
-CH₂-O-C₄H₈-O-CH₂
-C(CH₂OH)₂-
-CH(CH₂OH)-
-CH₂-O-C₆H₄-O-C₆H₄-O-CH₂-
-CHOH-CHOH-CHOH-CHOH-
-CH₂-O-CH₂-C(CH₂OH)₂-CH₂-O-CH₂-
-CH₂-CH(CH₂OCH₃)-CH₂-
-CH(OCH₃)-
-CH₂-O-CH₂-C₆H₄-CH₂-O-CH₂-

Im Gegensatz zu den Substraten III bis VI, die - verglichen mit dem Edukt II - äquimolar bis in beliebigem Überschuß, vorzugsweise mit 1,05 bis 2,0 Äquivalenten, umgesetzt werden, wird das Substrat VII in einem Unterschuß von 0,5 bis 0,3 Äquivalenten eingesetzt.

Im Substrat VII gegebenenfalls vorhandene Hydroxygruppen liegen vorzugsweise in geschützter Form, wie oben im Falle des Substrats III beschrieben, vor.

Das vorstehend beschriebene erfindungsgemäße Verfahren zeichnet sich durch hohe Ausbeuten, geringe Zahl an Reaktionsstufen, große Variationsbreite an gewünschten Substituenten R, problemloses Durchführen von Großansätzen (up-scaling), zum Teil durch möglichen Verzicht auf Lösungsmittel sowie durch problemloses Reinigen der Endprodukte aus.

Die nachfolgenden Beispiele dienen zur näheren Erläuterung des Erfindungsgegenstandes.

**Umsetzungen mit Verbindungen der allgemeinen Formel III:**

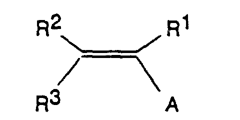

### Beispiel 1

### a) Gemisch aus 1-und 4-Formamido-10-[(2-Ethoxycarbonyl)-ethyl]-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel teilweise ab. Anschließend tropft man unter einer Stickstoffatmosphäre 13,94 g (139,2 mmol) Acrylsäureethylester zu und erwärmt langsam (innerhalb 30 Minuten) auf 80'C. Man rührt 12 Stunden bei dieser Temperatur. Man kühlt im Eisbad auf 0°C und gibt eine Mischung aus 150 ml Ethanol/20 ml Wasser zu. Dann wird 30 Minuten bei Raumtemperatur gerührt. Man dampft im Vakuum zu Trockene ein und chromatographiert den Rückstand an Kieselgel (Laufmittel= Ethanol/konz. aqu. Ammoniak= 10/1). Nach Eindampfen der Hauptfraktionen erhält man 31,71 g (91 % d. Th.) eines gelblichen Öls.

| Analyse (bezogen auf wasserfreie Substanz): | | | |
|---|---|---|---|
| ber. | C 55,98 | H 9,39 | N 18,65 |
| gef. | C 55,91 | H 9,43 | N 18,59 |

### b) 10-(2-Carboxyethyl)-1,4,7,10-tetraazacyclododecan

Zu 31,0 g (103,2 mmol) der Titelverbindung aus Beispiel 1a in 150 ml Ethanol/150 ml Wasser gibt man 46,32 g (825,6 mmol) Kaliumhydroxid und kocht 12 Stunden unter Rückfluß. Man kühlt im Eisbad auf 0°C. Es wird mit 6N Salzsäure auf pH 6 eingestellt und anschließend im Vakuum eingedampft. Der Rückstand wird mit einer Mischung aus 300 ml Methanol/50 ml Methylenchlorid extrahiert und vom Kaliumchlorid abfiltriert. Das Filtrat wird im Vakuum eingedampft und an einer Reversed-Phase Säule gereinigt (RP 18/Laufmittel: Gradient aus Tetrahydrofuran/Wasser).
Ausbeute: 23,02 g (87 % d. Th.) eines gelblichen, zähen Öls, das nach kurzer Zeit erstarrt
Analyse (bezogen auf wasserfreie Substanz):

| Analyse (bezogen auf wasserfreie Substanz): | | | |
|---|---|---|---|
| ber. | C 56,23 | H 9,44 | N 21,86 |
| gef. | C 56,17 | H 9,51 | N 21,83 |

### Beispiel 2

### 10-(2-Cyanoethyl)-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre werden 9,24 g (174,15 mmol) Acrylsäurenitril zugetropft und langsam auf 75°C erhitzt. Man rührt 9 Stunden bei dieser Temperatur. Es wird auf Raumtemperatur abgekühlt und eine Mischung aus 120 ml Methanol/30 ml Wasser zugegeben. Man rührt 10 Minuten bei Raumtemperatur. Anschließend gibt man 13,93 g (348,3 mmol) Natriumhydroxid zu und rührt 24 Stunden bei 40°C. Es wird im Vakuum zur Trockene eingedampft und der Rückstand 3 mal mit heißem Toluol (80°C) extrahiert. Die organische Phase wird über Kaliumhydroxid getrocknet und im Vakuum eingedampft.
Ausbeute: 23,28 g (89 % d. Th.) eines zartgelben Öles, das beim Stehenlassen kristallisiert.

| Analyse (bezogen auf wasserfreie Substanz): | | | |
|---|---|---|---|
| ber. | C 58,63 | H 10,29 | N 31,08 |
| gef. | C 58,57 | H 10,34 | N 30,96 |

### Beispiel 3

### 10-[(2-Phenyl-2-carboxy-)-ethyl]-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre werden 24,55 g (139,32 mol) 2-Phenyl-vinylsäureethylester zugetropft und langsam auf 130°C erhitzt. Man rührt 12 Stunden bei dieser Temperatur. Es wird auf Raumtemperatur abgekühlt und eine Mischung aus 150 ml Methanol/150 ml Wasser zugegeben. Anschließend wird 30 Minuten bei Raumtemperatur gerührt. Man gibt 52,11 g (928,8 mmol) Kaliumhydroxid zu und kocht 12 Stunden unter Rückfluß. Es wird im Eisbad auf 0°C gekühlt und mit konz. Salzsäure auf pH 7 gestellt, anschließend zur Trockene eingedampft. Der Rückstand wird in einer Mischung aus 250 ml Methanol/50 ml Methylenchlorid aufgenommen. Das ausgefallene Kaliumchlorid wird abfiltriert und das Filtrat im Vakuum eingeengt. Der Rückstand wird an Kieselgel chromatographiert (Laufmittel= Methyl-tert.-butylether/Methanol/konz. aqu. Ammoniak= 6/2/1).
Ausbeute: 28,27 g (76 % d. Th.) eines glasigen Feststoffes

| Analyse (bezogen auf wasserfreie Substanz): | | | |
|---|---|---|---|
| ber. | C 63,72 | H 8,81 | N 17,48 |
| gef. | C 63,64 | H 8,93 | N 17,37 |

### Beisplel 4

### 11-(2-Cyanoethyl)-1,4,8,11-Tetraazacyclotetradecan

Zu 20,0 g (99,83 mmol) 1,4,8,11 Tetraazacyclotetradecan in 200 ml absolutem Toluol gibt man 13,68 g (114,8 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre werden 6,36 g (119,8 mmol) Acrylsäurenitril zugetropft und langsam auf 75°C erhitzt. Man rührt 9 Stunden bei dieser Temperatur. Es wird auf Raumtemperatur abgekühlt und eine Mischung aus 120 ml Methanol/30 ml Wasser zugegeben. Man rührt 10 Minuten bei Raumtemperatur. Anschließend gibt man 11,98 g (299,5 mmol) Natriumhydroxid zu und rührt 24 Stunden bei 40°C. Es wird im Vakuum zur Trockene eingedampft und der Rückstand 3 mal mit heißem Toluol (80°C) extrahiert. Die organische Phase wird über Kaliumhydroxid getrocknet und im Vakuum eingedampft.
Ausbeute: 21,75 g (86 % d. Th.) eines zartgelben Öls, das beim Stehenlassen kristallisiert

| Analyse (bezogen auf wasserfreie Substanz): | | | |
|---|---|---|---|
| ber. | C 61,62 | H 10,74 | N 27,64 |
| gef. | C 61,53 | H 10,84 | N 27,52 |

In analoger Weise werden zum Beispiel die in der nachfolgenden Tabelle aufgeführten Verbindungen hergestellt.

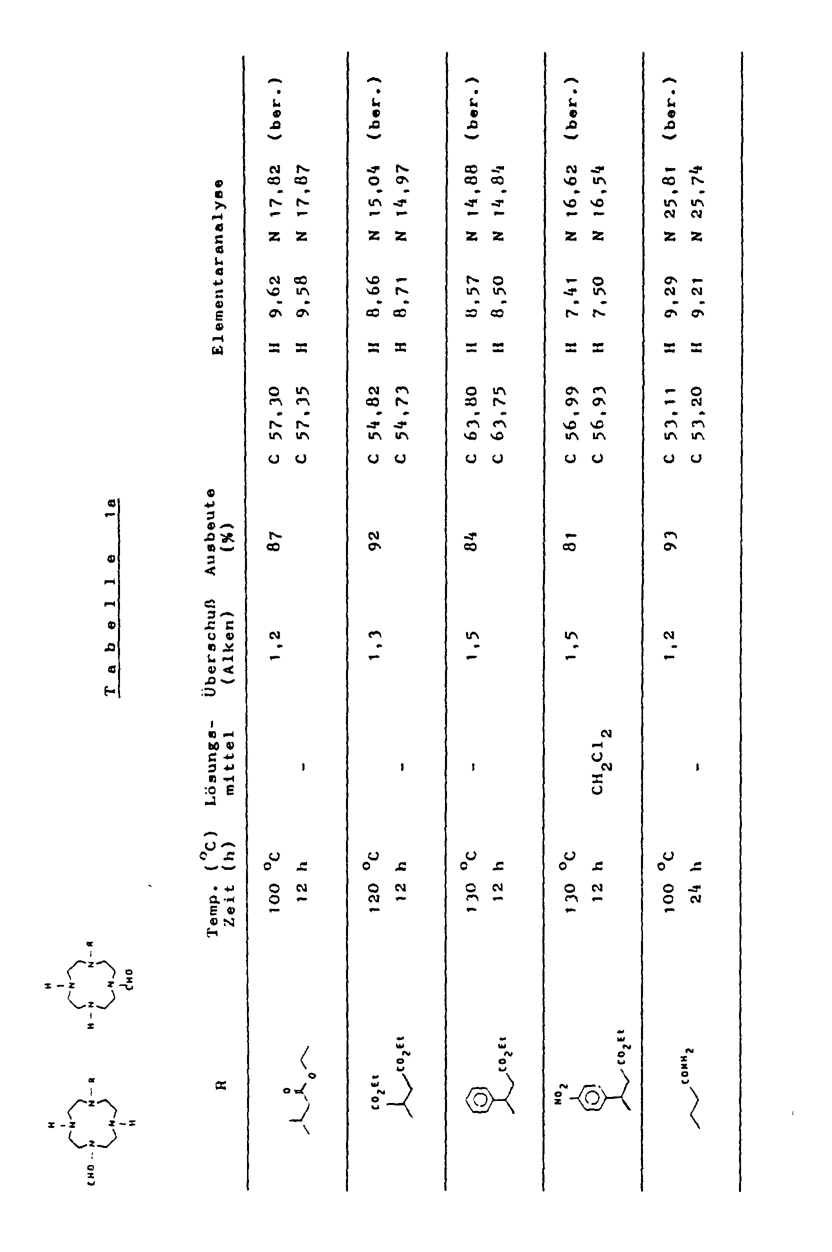

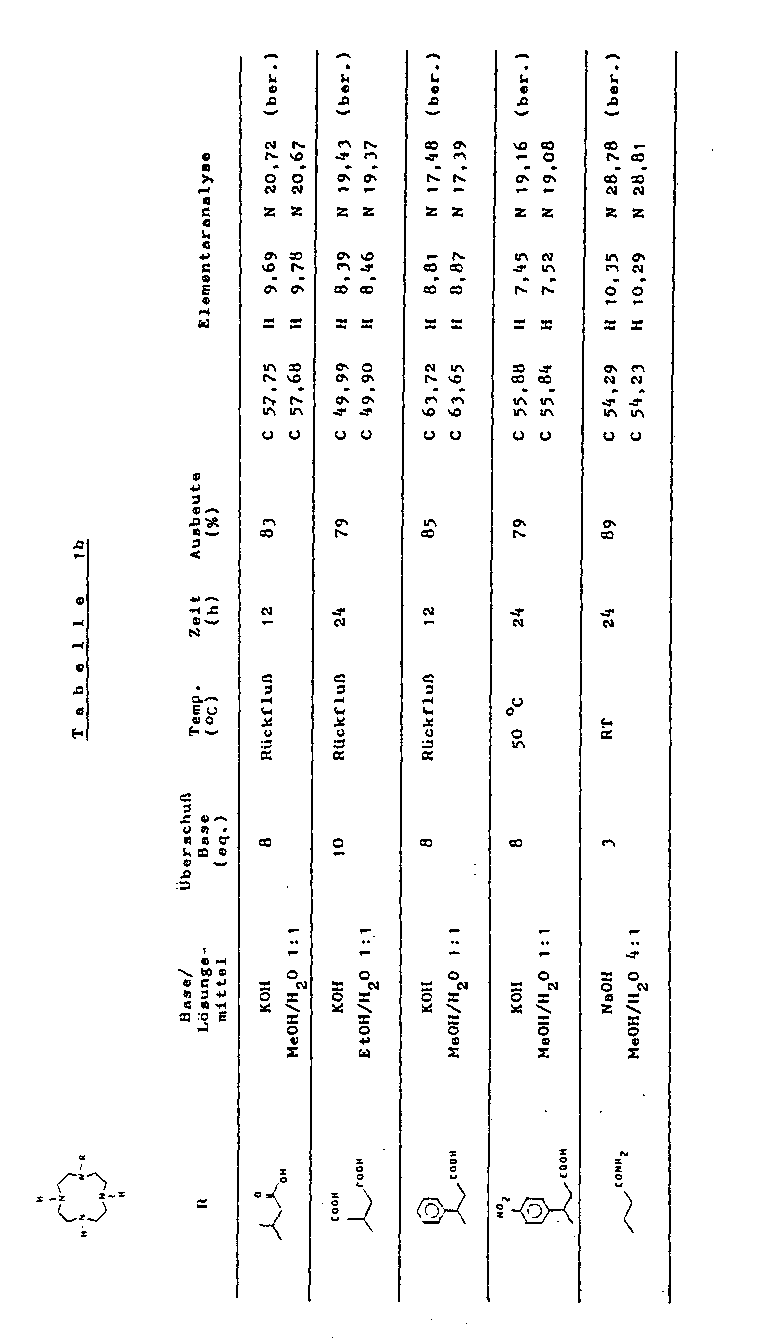

**Umsetzungen mit Verbindungen der allgemeinen Formel IV:**

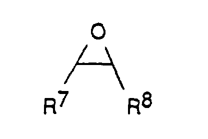

### Beispiel 5

### a) Gemisch aus 1-und 4-Formamido-10-(6-Hydroxy-2,2-dimethyl-1,3-dioxepan-5-yl)-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel teilweise ab. Anschließend tropft man unter einer Stickstoffatmosphäre 20,1 g (139,32 mmol) 4,4-Dimethyl-3,5,8-trioxabicyclo-(5.1.0)-octan zu und erwärmt langsam (1 Stunde) auf 130°C. Man rührt 12 Stunden bei 120°C. Es wird auf Raumtemperatur abgekühlt und eine Mischung aus 120 ml Methanol/30 ml Wasser zugegeben. Anschließend wird eine Stunde bei Raumtemperatur gerührt. Man dampft im Vakuum ein und chromatographiert den Rückstand an Kieselgel (Laufmittel= Methyl-tert.-butylether/Methanol/aqu. konz. Ammoniaks= 15/5/1). Nach Eindampfen der Hauptfraktionen erhält man 36,39 g (91 % d. Th.) eines zartgelben, zähen Öls, das beim Stehenlassen kristallisiert.

| Analyse (bezogen auf wasserfreie Substanz): | | | |
|---|---|---|---|
| ber. | C 55,79 | H 9,36 | N 16,27 |
| gef. | C 55,82 | H 9,29 | N 16,20 |

### b) 10-(6-Hydroxy-2,2-dimethyl-1,3-dioxepan-5-yl)-1,4,7,10-tetraazacyclododecan

Zu 35,0 g (101,6 mmol) der Titelverbindung aus Beispiel 5a in 200 ml Methanol/50 ml Wasser gibt man 57,0 g (1,02 mol) Kaliumhydroxid und kocht 5 Stunden unter Rückfluß. Man dampft im Vakuum zur Trockene ein und extrahiert den Rückstand 3 mal mit 200 ml heißem (80°C) Toluol. Die organische Phase wird über Kaliumhydroxid getrocknet und im Vakuum eingedampft.
Ausbeute: 31,5 g (98 % d. Th.) eines zartgelben, zähen Öls, das beim Stehenlassen fest wird

| Analyse (bezogen auf wasserfreie Substanz): | | | |
|---|---|---|---|
| ber. | C 56,93 | H 10,19 | N 17,71 |
| gef. | C 56,87 | H 10,25 | N 17,63 |

### Beispiel 6

### a) Gemisch aus 1-und 4-Formamidio-10-(2-hydroxypropyl)-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf 0°C abgekühlt. Man löst den Rückstand in 50 ml Toluol und füllt die Lösung in einen Autoklaven. Es werden 20,23 g (348,3 mmol) Propylenoxid zugesetzt und der Autoklav verschlossen. Anschließend wird 24 Stunden auf 100°C erwärmt. Man dampft im Vakuum zur Trockene ein und nimmt den Rückstand in einer Mischung aus 120 ml Methanol/30 ml Wasser auf. Dann wird eine Stunde bei Raumtemperatur gerührt. Es wird im Vakuum eingeengt und der Rückstand an Kieselgel chromatographiert. (Laufmittel= Methanol/Isopropanol/aqu. konz. Ammoniak= 10/5/1). Nach Eindampfen der Hauptfraktionen im Vakuum erhält man 26,7 g (89 % d. Th.) eines schwach gelbgefärbten Öls.

| Analyse (bezogen auf wasserfreie Substanz): | | | |
|---|---|---|---|
| ber. | C 55,79 | H 10,14 | N 21,69 |
| gef. | C 55,72 | H 10,19 | N 21,61 |

### b) 10-(2-Hydroxypropyl)-1,4,7,10-tetraazacyclododecan

Zu 26,0 g (100,63 mmol) der Titelverbindung aus Beispiel 6a in 250 ml Wasser gibt man 45,2 g (805,1 mmol) Kaliumhydroxid und kocht 5 Stunden unter Rückfluß. Man dampft im Vakuum zur Trockene ein und extrahiert den Rückstand 3 mal mit 200 ml heißem (80°C) Toluol. Die organische Phase wird über Kaliumhydroxid getrocknet und im Vakuum eingedampft.
Ausbeute: 22,02 g (95 % d. Th.) eines schwach gelblichen Öls, das nach kurzer Zeit erstarrt

| Analyse (bezogen auf wasserfreie Substanz): | | | |
|---|---|---|---|
| ber. | C 57,36 | H 11,38 | N 24,32 |
| gef. | C 57,30 | H 11,43 | N 24,28 |

### Beispiel 7

### 10-[2-Hydroxy-5-(2,2-dimethyl-1,3-dioxolan-4-yl)-4-oxa-pentyl]-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf 40°C abgekühlt. Unter einer Stickstoffatmosphäre werden 24,04 g (127,7 mmol) 2,2-Dimethyl-4-(2',3'-epoxy)-propoxy-methyl-1,3-dioxolan zugetropft und langsam (innerhalb einer Stunde) auf 110°C erhitzt. Man rührt 12 Stunden bei dieser Temperatur. Es wird auf Raumtemperatur abgekühlt und eine Mischung aus 120 ml Methanol/30 ml H₂O zugegeben. Dann wird 30 Minuten bei Raumtemperatur gerührt. Man gibt 65,1 g (1,16 mol) Kaliumhydroxid zu und kocht 5 Stunden unter Rückfluß. Anschließend wird im Vakuum eingeengt und der Rückstand 3 mal mit 200 ml heißem Toluol (80°C) extrahiert. Die vereinigten organischen Phasen werden über Kaliumhydroxid getrocknet und im Vakuum zur Trockene eingedampft. Man chromatographiert den Rückstand an Kieselgel (Laufmittel= Methanol/Wasser/aqu. konz. Ammoniak= 8/2/1). Die Hauptfraktionen werden zur Trockene eingedampft, der Rückstand in 500 ml heißem Toluol gelöst. Man filtriert vom Unlöslichen ab (Kieselgel) und dampft zur Trockene ein.
Ausbeute: 36,41 g (87 % d. Th.) eines zartgelben, zähen Öls

| Analyse (bezogen auf wasserfreie Substanz): | | | |
|---|---|---|---|
| ber. | C 56,64 | H 10,07 | N 15,54 |
| gef. | C 56,53 | H 10,13 | N 15,49 |

### Beispiel 8

### 10-[3-(4-Nitrophenoxy)-2-hydroxypropyl]-1,4,7,10-tetraazacyclododecan (als Tetra-Hydrochlorid)

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre wird eine Lösung aus 29,46 g (150,93 mmol) 4-(Nitrophenyl)-2,3-epoxypropylether in 100 ml Methylenchlorid zugetropft. Anschließend wird langsam auf 120°C erwärmt, wobei man das Methylenchlorid abdestilliert (gegen Ende unter reduziertem Druck). Es wird 12 Stunden bei 120°C gerührt. Man kühlt auf Raumtemperatur und gibt eine Mischung aus 160 ml Methanol/20 ml Wasser zu. Dann wird 30 Minuten bei Raumtemperatur gerührt. Es werden 50 ml konz. Salzsäure zugegeben und 12 Stunden unter Rückfluß erhitzt. Anschliessend wird im Vakuum zur Trockene eingedampft. Der Rückstand wird aus Methanol/Ether umkristallisiert.
Ausbeute: 48,27 g (81 % d. Th.) eines gelbgefärbten kristallinen Pulvers

| Analyse (berechnet auf Cl-freie Verbindung): | | | |
|---|---|---|---|
| ber. | C 55,57 | H 7,95 | N 19,06 |
| gef. | C 55,49 | H 8,03 | N 19,01 |

### Beispiel 9

### a) 11-[3-(4-Nitroxyphenoxy)-2-hydroxypropyl]-1,4,8,11 Tetraazacyclotetradecan (als Tetra-Hydrochlorid)

Zu 20,0 g (99,83 mmol) 1,4,8,11 Tetraazacyclotetradecan in 200 ml absolutem Toluol gibt man 13,68 g (114,8 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre wird eine Lösung aus 23,38 g (119,8 mmol) 4-Nitrophenyl-2,3-epoxypropylether in 100 ml Methylenchlorid zugetropft. Anschließend wird langsam auf 120°C erwärmt, wobei man das Methylenchlorid abdestilliert (gegen Ende unter reduziertem Druck). Es wird 12 Stunden bei 120°C gerührt. Man kühlt auf Raumtemperatur und gibt eine Mischung aus 150 ml Methanol/20 ml Wasser zu. Dann wird 30 Minuten bei Raumtemperatur gerührt. Es werden 100 ml konz. Salzsäure zugegeben und 12 Stunden unter Rückfluß erhitzt. Anschließend wird im Vakuum zur Trockene eingedampft. Der Rückstand wird aus Methanol/Ether umkristallisiert.
Ausbeute: 41,61 g (77 % d. Th.) eines gelblichen, kristallinen Pulvers

| Analyse (bezogen auf wasserfreie Substanz): | | | | |
|---|---|---|---|---|
| ber. | C 42,16 | H 6,89 | N 12,94 | Cl 26,20 |
| gef. | C 42,10 | H 6,93 | N 12,90 | Cl 26,08 |

### Beispiel 10

### a) Gemisch aus 1-und 4-und 8-Formamido-11-[2-hydroxy-2-(2,2-dimethyl-1,3-dioxolan-4-yl)-ethyl]-1,4,8,11-tetraazacyclotetradecan

Zu 20,0 g (99,83 mmol) 1,4,8,11 Tetraazacyclotetradecan in 200 ml absolutem Toluol gibt man 13,68 g (114,8 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf Raumtemperatur abgekühlt. Unter einer Stickstoffatmosphäre werden 17,27 g (119,8 mmol) 2-(2,2-Dimethyl-1,3-dioxolan-4-yl)-ethylenoxid zugetropft und dann langsam auf 130°C erwärmt. Man rührt 12 Stunden bei dieser Temperatur. Es wird auf 0°C abgekühlt und eine Mischung aus 160 ml Methanol/40 ml Wasser zugegeben, anschließend 1 Stunde bei Raumtemperatur gerührt. Man dampft im Vakuum zur Trockene ein und chromatographiert den Rückstand an Kieselgel (Laufmittel= Methyl-tert.-butylether/Methanol/konz. aqu. Ammoniak= 15/5/1).
Ausbeute: 33,1 g (89 % d. Th.) eines zartgelben, zähen Öls

| Analyse (bezogen auf wasserfreie Substanz): | | | |
|---|---|---|---|
| ber. | C 58,04 | H 9,74 | N 15,04 |
| gef. | C 58,13 | H 9,61 | N 14,92 |

### b) 11-[2-Hydroxy-2-(2,2-dimethyl-1,3-dioxoian-4-yl}-ethyl]-1,4,8,11-tetraazacyclotetradecan

Zu 32,0 g (87,22 mmol) der Titelverbindung aus Beispiel 10a in 200 ml Methanol/100 ml Wasser gibt man 39,15 g (698 mmol) Kaliumhydroxid und kocht 5 Stunden unter Rückfluß. Man dampft im Vakuum zur Trockene ein und extrahiert den Rückstand 3 mal mit 200 ml heißem Toluol (80°C). Die organische Phase wird über Kaliumhydroxid getrocknet und im Vakuum eingedampft.
Ausbeute: 28,85 g (96 % d. Th.) eines zartgelben, zähen Öls, das nach kurzer Zeit erstarrt

| Analyse (bezogen auf wasserfreie Substanz): | | | |
|---|---|---|---|
| ber. | C 59,27 | H 10,53 | N 16,26 |
| gef. | C 59,18 | H 10,61 | N 16,17 |

In analoger Weise werden zum Beispiel die in der nachfolgenden Tabelle aufgeführten Verbindungen hergestellt.

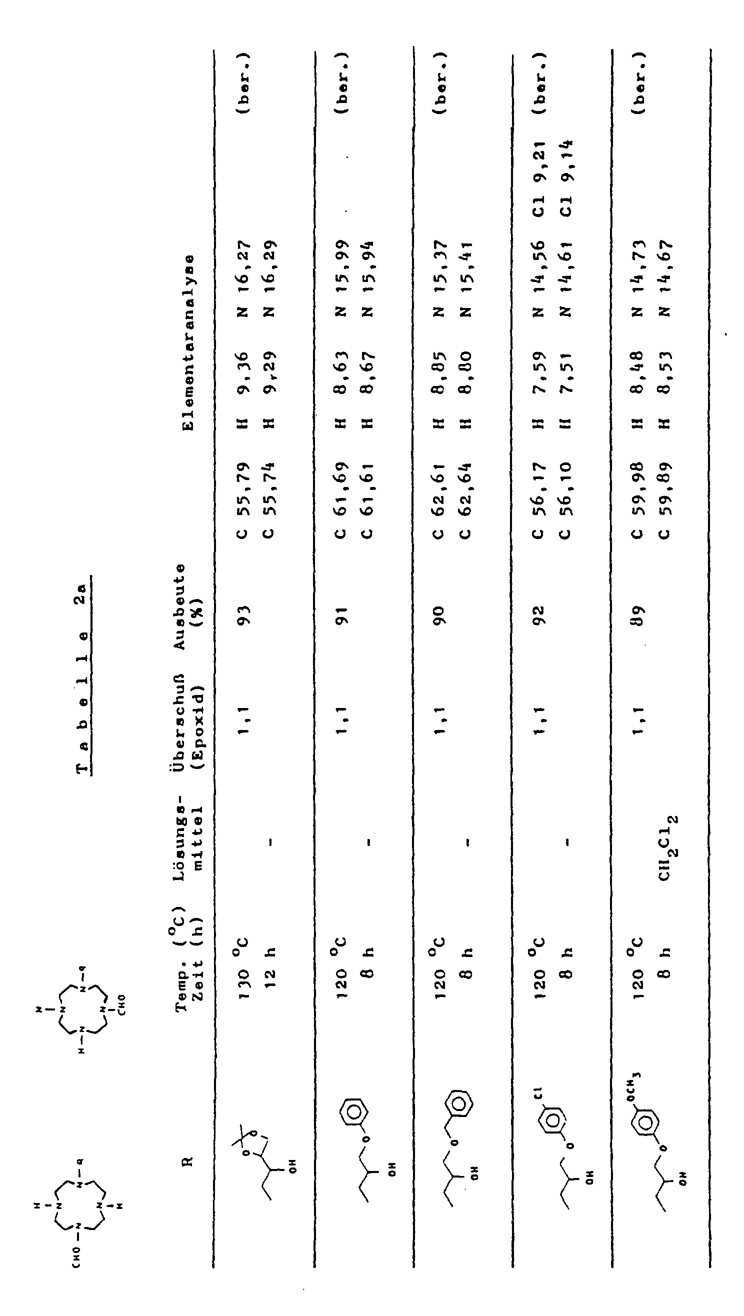

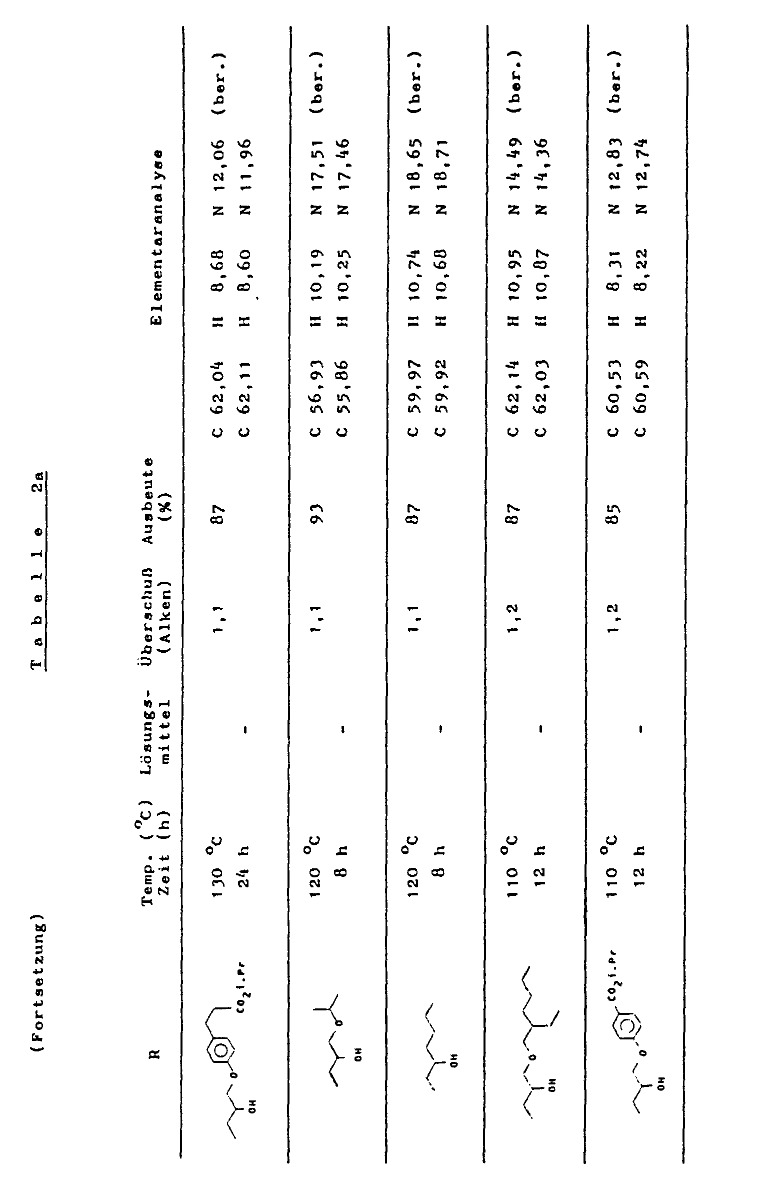

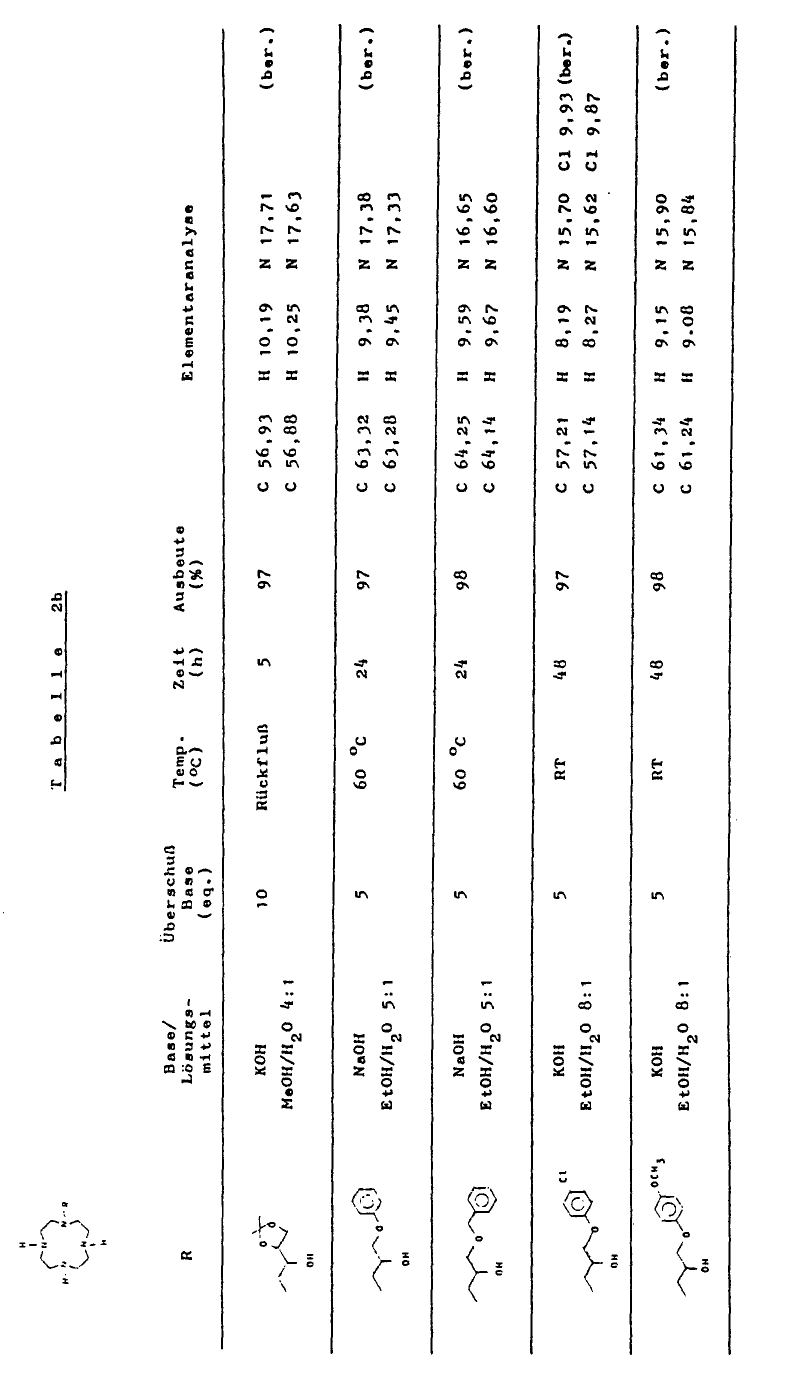

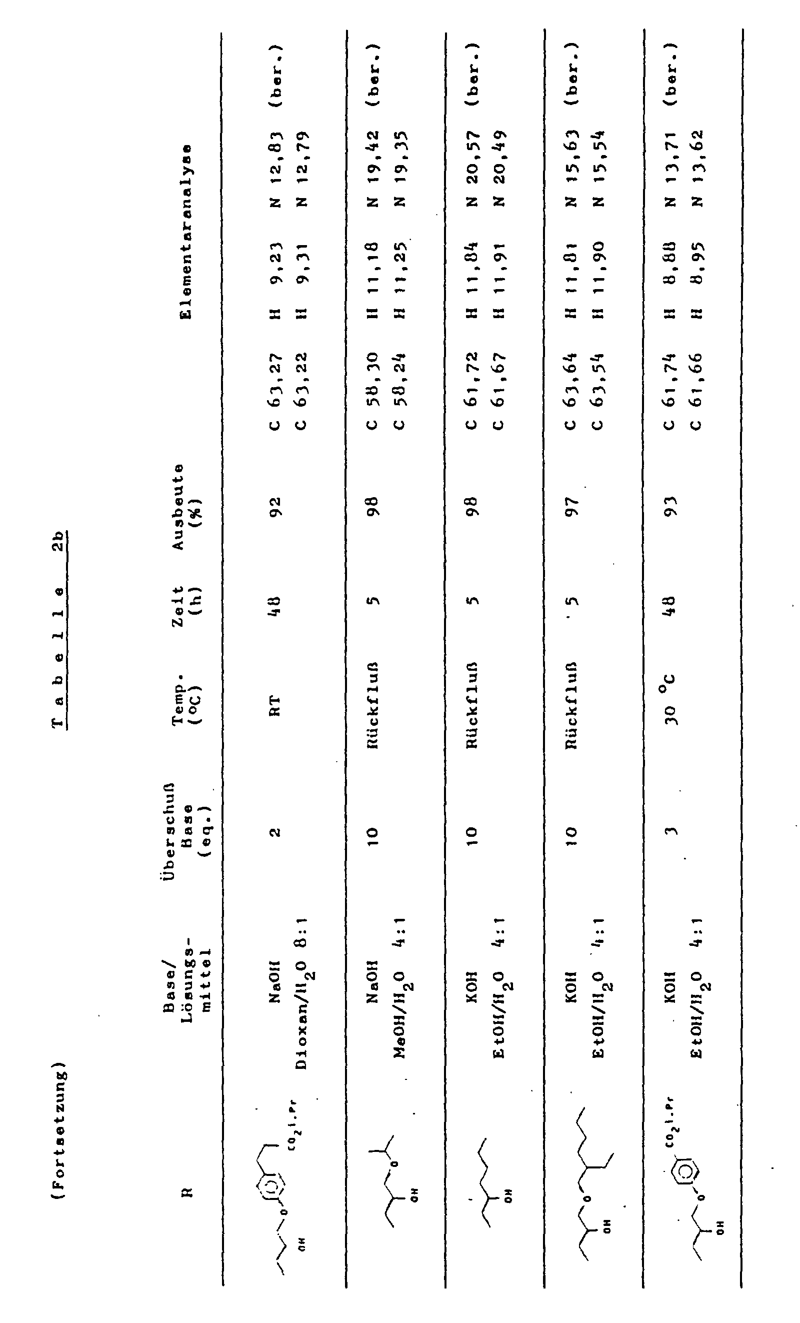

**Umsetzungen mit Verbindungen der allgemeinen Formel V:**

R⁹-N = C = X

### Beispiel 11

### 10-(N-Phenylcarbamoyl)-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf 0°C abgekühlt. Unter einer Stickstoffatmosphäre werden 16,6 g (139,32 mmol) Phenylisocyanat zugetropft und langsam auf 100°C erwärmt. Man rührt 12 Stunden bei dieser Temperatur. Es wird auf Raumtemperatur abgekühlt und eine Mischung aus 160 ml Ethanol/40 ml Wasser zugegeben. Dann wird 10 Minuten bei Raumtemperatur gerührt. Anschließend setzt man 18,58 g (464,4 mmol) Natriumhydroxid zu und rührt 24 Stunden bei 40°C. Man engt im Vakuum zur Trockene ein und nimmt den Rückstand in 400 ml Wasser auf. Die wässrige Phase wird 5 mal mit 200 ml Methylenchlorid extrahiert, die organische Phase über Magnesiumsulfat getrocknet und im Vakuum eingeengt. Der Rückstand wird an Kieselgel chromatographiert (Laufmittel= Methyl.-tert.-butylether/Methanol/konz. aqu. Ammoniak= 6/3/1).
Ausbeute: 29,1 g (86 % d. Th.) eines zartgelben Feststoffes

| Analyse (bezogen auf wasserfreie Substanz): | | | |
|---|---|---|---|
| ber. | C 61,83 | H 8,65 | N 24,03 |
| gef. | C 61,71 | H 8,72 | N 23,94 |

In analoger Weise werden zum Beispiel die in der nachfolgenden Tabelle aufgeführten Verbindungen hergestellt.

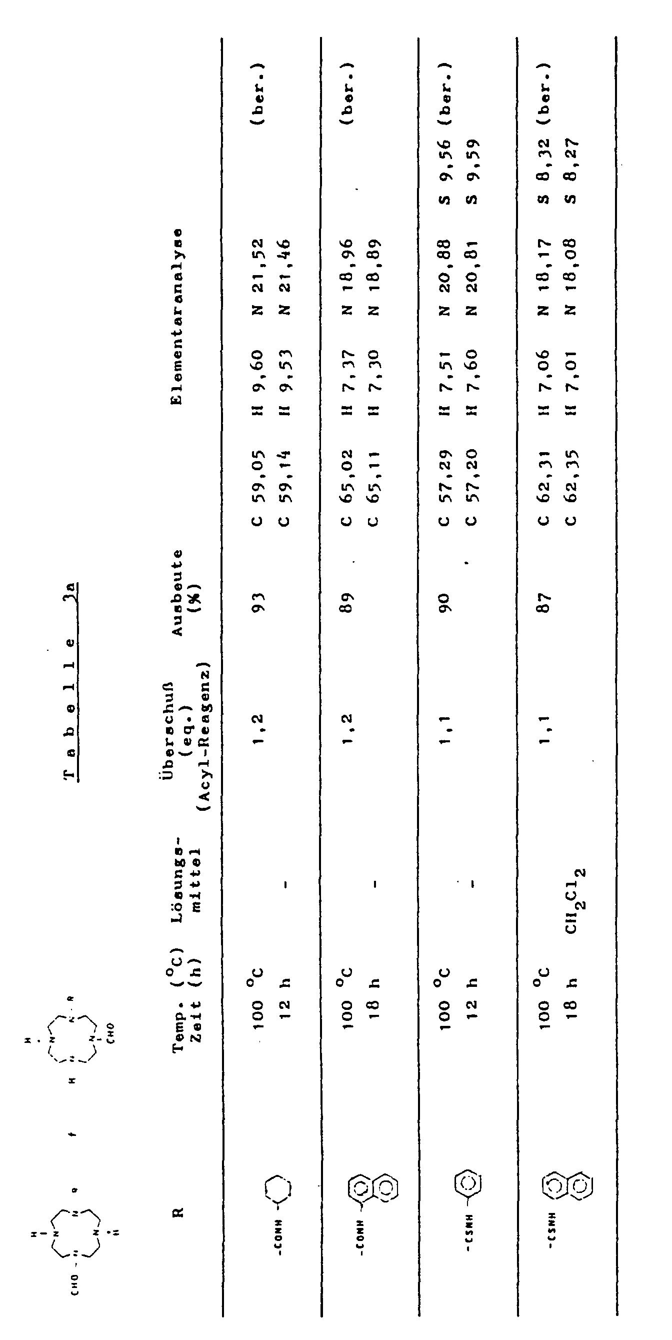

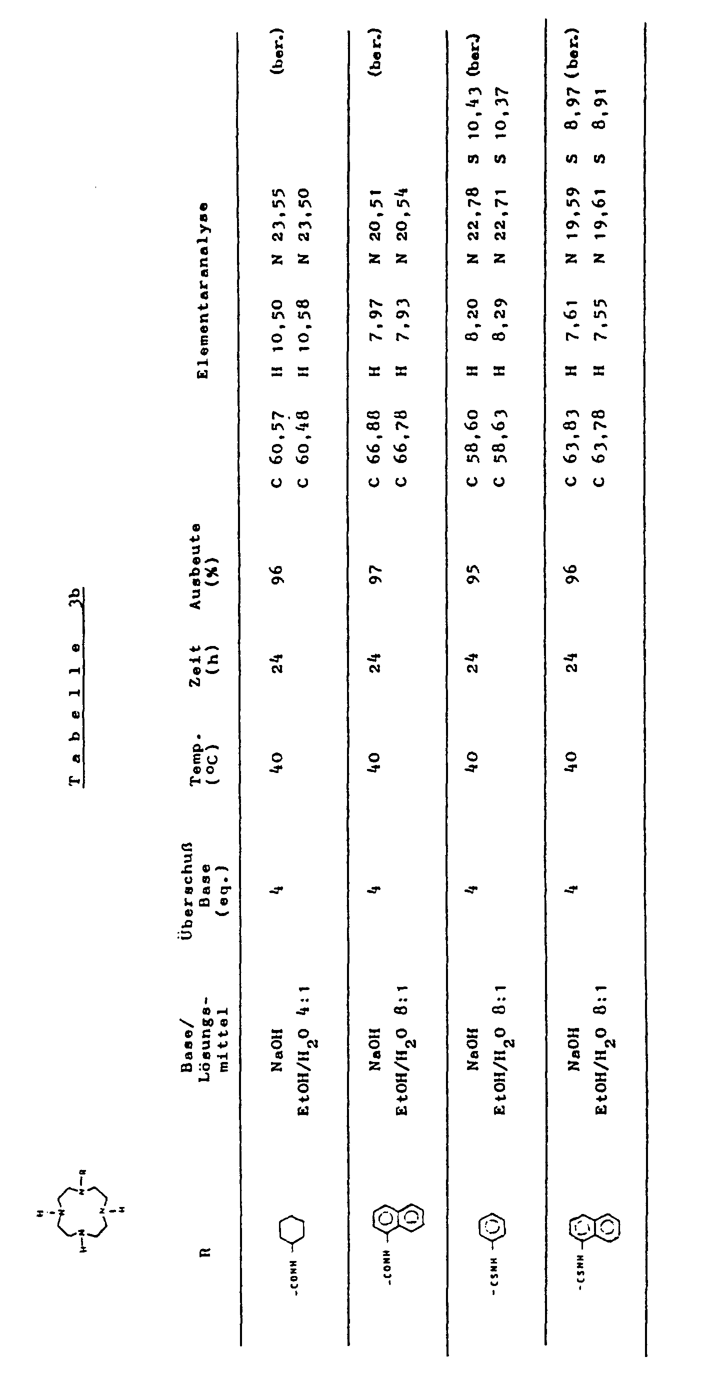

**Umsetzungen mit Verbindung der allgemeinen Formel VI:**

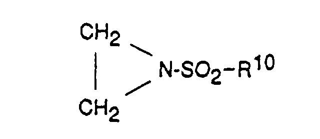

### Beispiel 12

### a) Gemisch aus 1-und 4-Formamido-10-[2-(p-Tolylsulfonylamino)-ethyl]-1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf 0°C abgekühlt. Unter einer Stickstoffatmosphäre wird eine Lösung aus 25,19 g (127,71 mmol) p-Tolylsulfonylaziridin in 100 ml Toluol zugetropft und anschließend 8 Stunden bei 80°C gerührt. Man dampft im Vakuum zur Trockene ein und nimmt den Rückstand in einer Mischung aus 180 ml Ethanol/30 ml Wasser auf. Dann wird 30 Minuten bei Raumtemperatur gerührt. Anschließend wird zur Trockene eingeengt. Der Rückstand wird an Kieselgel chromatographiert (Laufmittel= Methyltert.-butylester/Methanol/konz. aqu. Ammoniak= 6/2/1).
Ausbeute: 40,61 g (88 % d. Th.) eines glasigen Feststoffes

| Analyse (bezogen auf wasserfreie Substanz): | | | | |
|---|---|---|---|---|
| ber. | C 54,38 | H 7,86 | N 17,62 | S 8,06 |
| gef. | C 54,31 | H 7,93 | N 17,58 | S 7,99 |

### b) 10-[2-(p-Tolylsulfonylamino)-ethyl]-1,4,7,10-tetraazacyclodecan

Zu 40,0 g (100,62 mmol) der Titelverbindung 9a in 180 ml Ethanol/30 ml Wasser gibt man 20,12 g (503 mmol) Natriumhydroxid und kocht 12 Stunden unter Rückfluß. Man dampft zur Trockene ein und nimmt den Rückstand in 100 ml Wasser auf. Der pH-Wert der Lösung wird durch Zugabe von 6N-Salzsäure auf pH 10 gebracht. Anschließend wird 2 mal mit 250 ml heißem Toluol (80°C) extrahiert. Die organische Phase wird über Magnesiumsulfat getrocknet und im Vakuum eingedampft.
Ausbeute: 36,07 g (97 % d. Th.) eines gelblichen, glasigen Feststoffes

| Analyse (bezogen auf wasserfreie Substanz): | | | | |
|---|---|---|---|---|
| ber. | C 55,26 | H 8,46 | N 18,95 | S 8,68 |
| gef. | C 55,21 | H 8,52 | N 18,90 | S 8,59 |

### c) Gemisch aus 1-und 4-Formamido-10-[2-(methylsulfonylamino)-ethyl]-1,4,7,10-tetraazacyclododecan

Analog zu Beispiel 12a kann Methylsulfonylaziridin anstelle von p-Tolylsulfonylaziridin eingesetzt werden.
Ausbeute: 89 % d. Th.

| Analyse (bezogen auf wasserfreie Substanz): | | | | |
|---|---|---|---|---|
| ber. | C 44,84 | H 8,47 | N 21,79 | S 9,97 |
| gef. | C 44,76 | H 8,53 | N 21,73 | S 9,90 |

### d) 10-[2-(methylsulfonylamino)-ethyl]-1,4,7,10-tetraazacyclododecan

Analog zu Beispiel 12b kann die Titelverbindung aus Beispiel 12c anstelle der Titelverbindung 12a eingesetzt werden.
Ausbeute: 96 % d. Th.

| Analyse (bezogen auf wasserfreie Substanz): | | | | |
|---|---|---|---|---|
| ber. | C 45,03 | H 9,27 | N 23,87 | S 10,93 |
| gef. | C 44,96 | H 9,34 | N 23,98 | S 10,85 |

**Umsetzungen mit Verbindungen der allgemeinen Formel VII:**

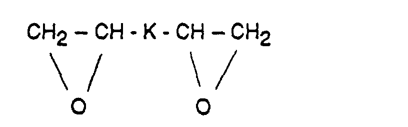

### Beispiel 13

### a) Gemisch der Bis-Formamide von 1,1'-(2,6-Dihydroxy-4-oxa-1,7-heptyl)-bis-[1,4,7,10-tetraazacyclododecan

Zu 20,0 g (116,1 mmol) 1,4,7,10 Tetraazacyclododecan in 200 ml absolutem Toluol gibt man 15,9 g (133,5 mmol) Dimethylformamid-dimethylacetal (unter Stickstoff). Man erwärmt langsam zum Rückfluß und destilliert dabei das Lösungsmittel ab. Anschließend wird unter reduziertem Druck eingeengt. Der Rückstand wird auf 40°C abgekühlt. Unter einer Stickstoffatmosphäre tropft man 7,25 g (55,7 mmol) Bis-[2,3-epoxypropyl]-ether zu und erwärmt langsam auf 120°C. Man rührt 24 Stunden bei dieser Temperatur. Es wird auf Raumtemperatur abgekühlt und eine Mischung aus 200 ml Methanol/100 ml Wasser zugegeben. Anschließend wird eine Stunde bei Raumtemperatur gerührt. Man dampft zur Trockene ein und chromatographiert den Rückstand an Kieselgel (Laufmittel= Methanol/Isopropanol/konz. aqu. Ammoniak= 8/2/1).
Ausbeute: 18,63 g (63 % d. Th.) eines glasigen Feststoffes

| Analyse (bezogen auf wasserfreie Substanz): | | | |
|---|---|---|---|
| ber. | C 54,32 | H 9,50 | N 21,11 |
| gef. | C 54,25 | H 9,57 | N 21,18 |

### b) 1,1'-(2,6-Dihydroxy-4-oxa-1,7-heptyl)-bis-(1,4,7,10-tetraazacyclododecan)

Zu 18,0 g (33,92 mmol) der Titelverbindung aus Beispiel 13a in 200 ml Methanol/100 ml Wasser gibt man 28,55 g (508,7 mmol) Kaliumhydroxid und kocht 2. Stunden unter Rückfluß. Man dampft im Vakuum zur Trockene ein und extrahiert den Rückstand 3 mal mit 200 ml heißem Toluol (80°C). Die organische Phase wird über Kaliumhydroxid getrocknet und im Vakuum eingedampft.
Ausbeute: 15,62 g (97 % d. Th.) eines zartgelben, zähen Öls, das beim Stehenlassen erstarrt.

| Analyse (bezogen auf wasserfreie Substanz): | | | |
|---|---|---|---|
| ber. | C 55,67 | H 10,62 | N 23,61 |
| gef. | C 55,61 | H 10,68 | N 23,56 |

In analoger Weise werden zum Beispiel die in der nachfolgenden Tabelle aufgeführten Verbindungen hergestellt.

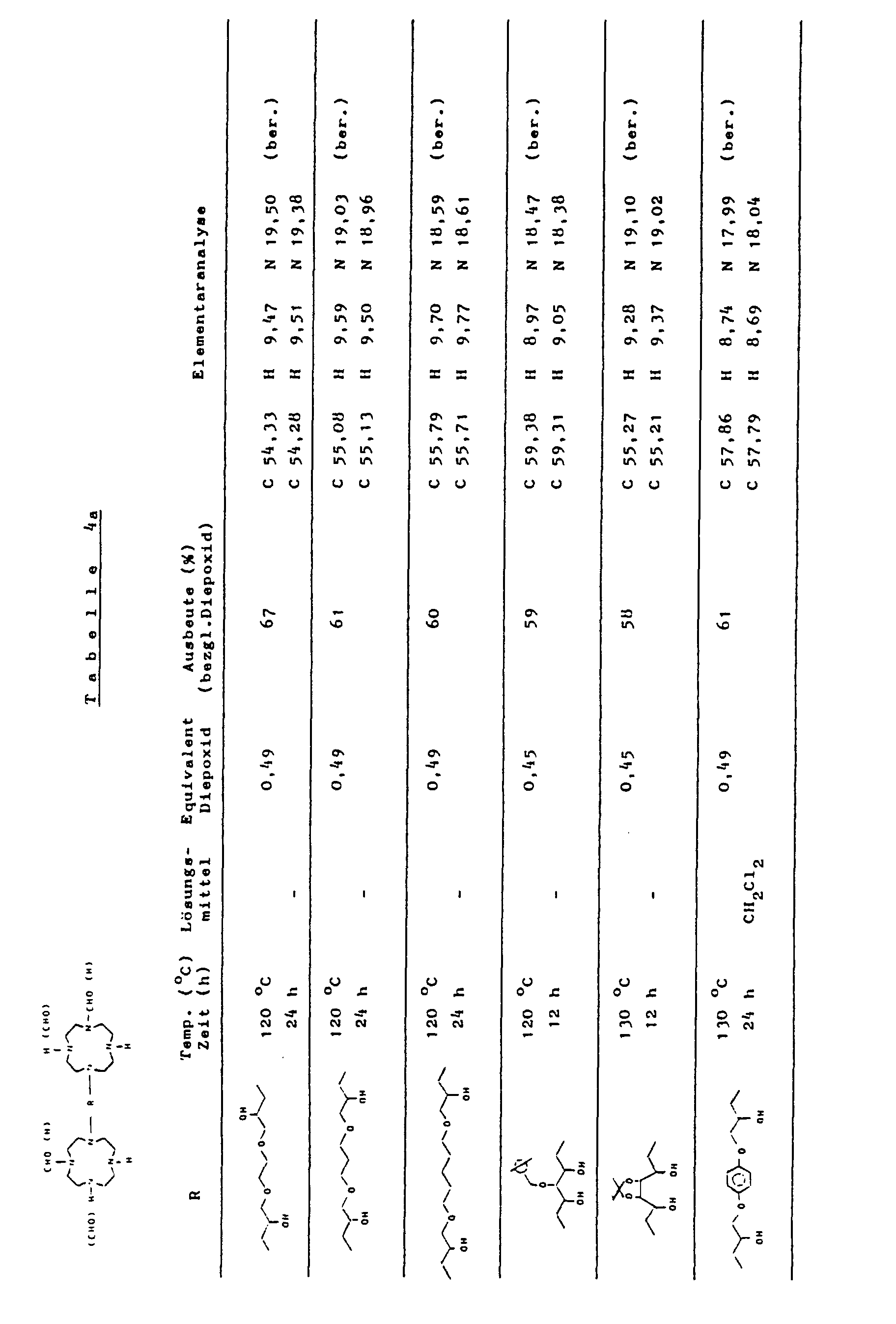

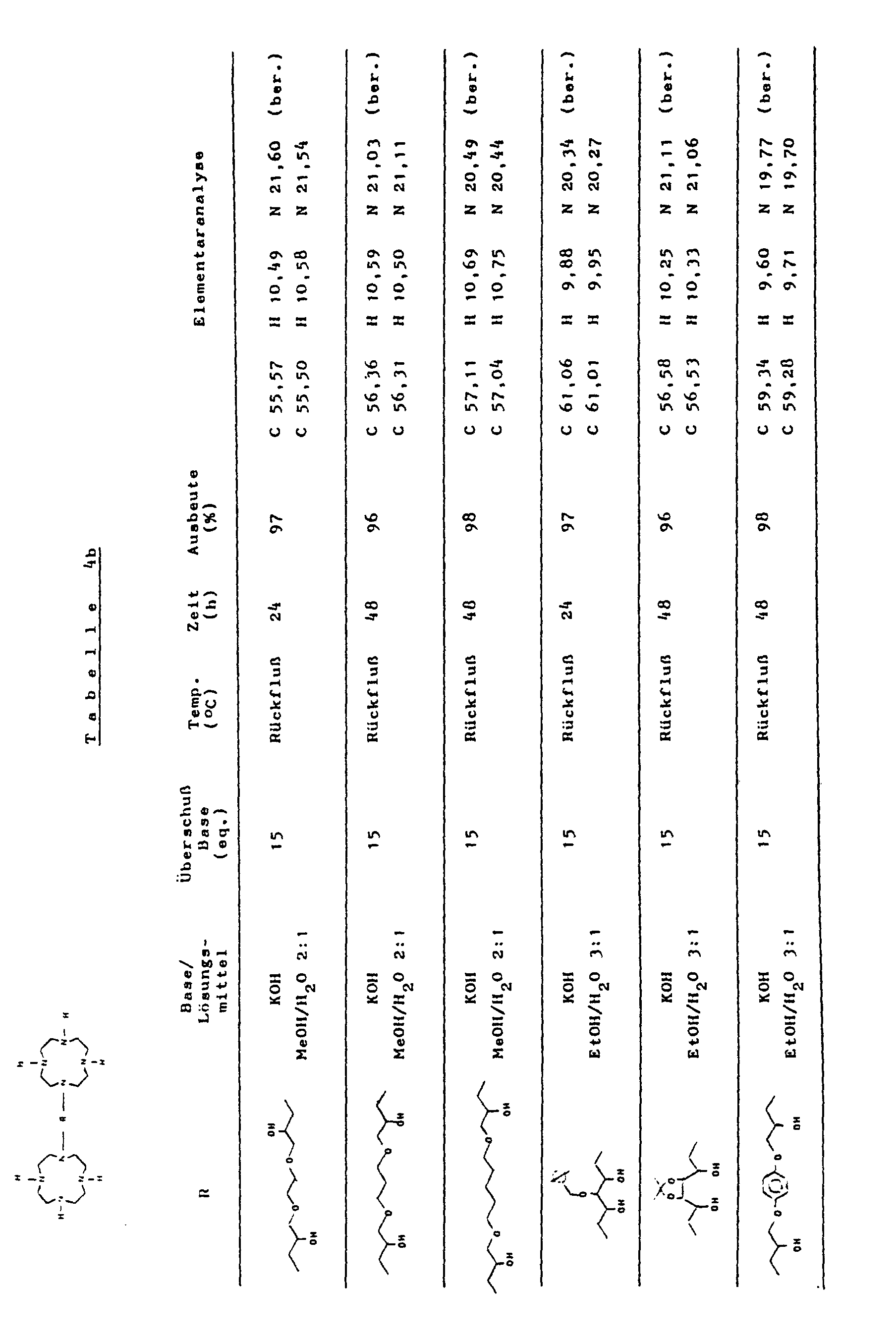

## Patentansprüche

1. Verfahren zur Herstellung von mono-N-substituierten Tetraazacyclododecanund -tetradecan-Derivaten der allgemeinen Formel I 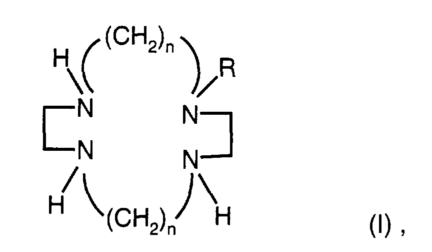 worin
n für die Ziffern 2 oder 3,
R für eine Gruppe -CR²R³-CHR¹-A, -CHR⁷-CH(OH)-R⁸, -C(X)NHR⁹, -(CH₂)₂-NHSO₂R¹⁰ oder für ein über -CH₂-CH(OH)-K-CH(OH)-CH₂- gebundenes zweites Tetraazacyclododecan- oder -tetradecan-Molekül steht
worin
R¹ für ein Wasserstoffatom, eine geradkettige oder cyclische C₁-C₆-Alkyl-, eine Phenyl- oder Benzylgruppe - wobei die Phenylgruppe jeweils durch 1 bis 2 Chlor-, Brom-, Nitro-, C₁-C₇-Alkoxy- oder Aralkoxy- oder CO₂R⁴-reste mit R⁴ in der Bedeutung eines Wasserstoffatoms, einer C₁-C₆-Alkyl-, Phenyl- oder Benzylgruppe substituiert sein kann - steht,
R² und R³ unabhängig voneinander jeweils für R¹ oder eine CO₂R⁴-Gruppe stehen,
A für einen CN-, CO₂R⁴- oder 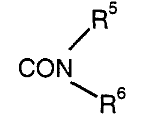 -Rest steht,
worin
R⁵ und R⁶ unabhängig voneinander jeweils für ein Wasserstoffatom, einen gegebenenfalls durch 1 bis 5 Hydroxygruppe(n) oder 1 bis 2 CO₂R⁴-Reste substituierten gesättigten, ungesättigten, gerad- oder verzweigtkettigen oder cyclischen, gegebenenfalls durch 1 bis 8 Sauerstoffatom(e) oder 1 bis 3 Phenylen- oder Phenylenoxygruppe(n) unterbrochenen Kohlenwasserstoffrest mit bis zu 16 C-Atomen, für gegebenenfalls durch 1 bis 3 Hydroxy- oder C₁-C₆-Alkoxygruppen substituierte Phenyl- oder Benzylreste oder R⁵ und R⁶ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3, gegebenenfalls durch 1 bis 3 Hydroxyreste substituierte C₁-C₆-Alkylreste, wobei gegebenenfalls vorhandene Hydroxy- und/oder Carboxylgruppen gegebenenfalls geschützt sind,
R⁷ und R⁸ unabhängig voneinander jeweils für ein Wasserstoffatom, einen gegebenenfalls durch 1 bis 10 Sauerstoffatom(e), eine Phenylen-, Phenylenoxy- oder Phenylendioxygruppe unterbrochenen C₁-C₂₀-Alkylrest, der gegebenenfalls durch 1 bis 3 C₁-C₆-Alkyl-, 1 bis 3 Trifluormethyl, 1 bis 7 Hydroxy-, 1 bis 3 C₁-C₇-Alkoxy- oder -Aralkoxy-, 1 bis 2 CO₂R⁴- und/oder 1 bis 2, gegebenenfalls durch 1 bis 2 Chlor-, Brom-, Nitro- oder C₁-C₆-Alkoxyreste substituierte Phenoxy- oder Phenylgruppen substituiert ist, stehen, wobei die gegebenenfalls vorhandenen Hydroxyreste gegebenenfalls in geschützter Form vorliegen,
X für ein Sauerstoff- oder Schwefelatom und
R⁹ für eine Phenyl-, 1- oder 2-Naphthyl- oder geradkettige oder cyclische C₁-C₆-Alkylgruppe,
R¹⁰ für eine C₁-C₆-Alkyl-, -CF₃- oder eine gegebenenfalls durch einen C₁-C₆-Alkyl-, Chlor-, Brom- oder Nitrorest substituierte Phenylgruppe, und
K für eine gegebenenfalls durch 1 bis 6 Sauerstoffatome, 1 bis 2 Phenylen-, Phenylenoxy- oder Phenylendioxygruppen unterbrochene, gegebenenfalls durch 1 bis 6 Hydroxy-, Hydroxyalkyl- oder 1 bis 6 C₁-C₇-Alkoxy-, Benzyloxy- oder Aralkoxygruppen substituierte C₀-C₁₆-Alkylenkette stehen, wobei Carboxyl- und Hydroxygruppen gegebenenfalls in geschützter Form vorliegen, **dadurch gekennzeichnet, daß** man die aus 1,4,7,10-Tetraazacyclododecan bzw. 1,5,8,11-Tetraazacyclotetradecan erhaltenen Verbindungen der allgemeinen Formel II 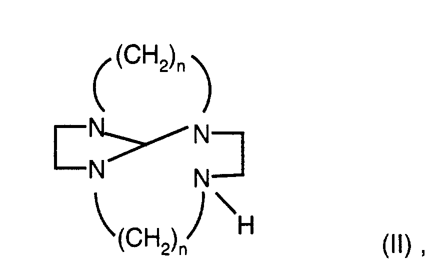
mit einem entsprechenden Edukt der allgemeinen Formel 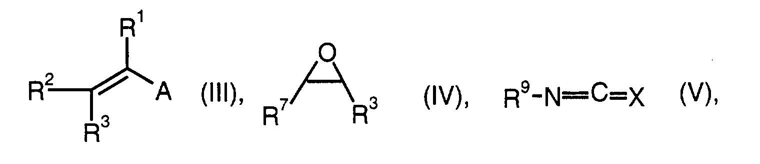 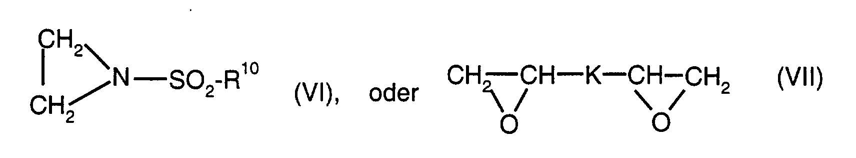 worin
R¹-R¹⁰, A, X und K die oben angegebene Bedeutung haben,
mit oder ohne Lösungsmittel bei 0 bis 220 °C, innerhalb von 1 bis 48 Stunden, gegebenenfalls bei einem Druck bis zu 100 atm. umsetzt, anschließend das so erhaltene Reaktionsgemisch nach Abkühlung auf -20 °C bis 80 °C, mit einem Gemisch Wasser/organisches Lösungsmittel, ausgewählt aus Methanol, Ethanol, iso-Propanol, Tetrahydrofuran oder Dioxan, versetzt und 0,5 bis 12 Stunden, bei -20 °C bis Raumtempratur rührt, dann die so gebildeten - gewünschtenfalls zu isolierenden - eine Formylgruppe an einem Stickstoffatom tragenden Zwischenprodukte durch Zugabe einer anorganischen Base, ausgewählt aus Lithium-, Natrium-, Kalium-, Barium- oder Calciumhydroxid oder einer Mineralsäure, ausgewählt aus Salz- Schwefel- oder Bromwasserstoffsäure bei 0 bis 150 °C, innerhalb von 1 bis 72, unter Rühren- gegebenenfalls gefolgt von anschließender Schutzgruppenentfernung in an sich üblicher Weise - zum Endprodukt der Formel I umsetzt und anschließend in an sich bekannter Weise isoliert.

2. Verfahren gemäß Anspruch 1 zur Herstellung von Verbindungen der allgemeinen Formel I mit
R in der Bedeutung einer 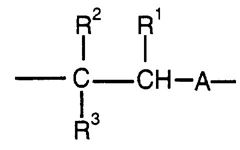 Gruppe,
worin
R¹, R², R³ und A die in Anspruch 1 angegebene Bedeutung haben,
**dadurch gekennzeichnet, daß** man Tetraazatricyclotridecan bzw. -pentadecan mit einem Edukt der allgemeinen Formel III 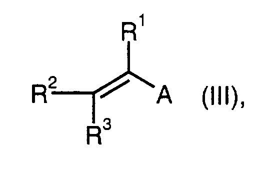 worin
R¹, R², R³ und A die oben angegebene Bedeutung haben, wobei gegebenenfalls vorhandene Hydroxy- und/oder Carboxylgruppen gegebenenfalls geschützt sind, mit oder ohne Lösungsmittel, wobei als Lösungsmittel aprotische Lösungsmittel ausgewählt aus Benzol, Toluol, Dichlormethan, Tetrahydrofuran, Dioxan, Acetonitril, Dimethylformamid, Hexan oder Ether verwendet werden, bei 0 °C bis 210 °C (wobei im Falle der höheren Umsetzungstemperatur das gegebenenfalls zum Lösen des zugesetzten Edukts der allgemeinen Formel III verwendete Lösungsmittel vorher im Vakuum abdestilliert worden ist), innerhalb von 12 bis 48 Stunden, umsetzt, anschließend das so erhaltene Reaktionsgemisch auf -20 °C bis 80 °C abkühlt, mit einem Gemisch Wasser/organisches Lösungsmittel ausgewählt aus Methanol, Ethanol, iso-Propanol, Tetrahydrofuran oder Dioxan versetzt und 0,5 bis 12 Stunden bei -20 °C bis Raumtemperatur rührt, dann das so gebildete - gewünschtenfalls zu isolierende - eine Formylgruppe an einem Stickstoffatom tragende Zwischenprodukt durch Zugabe einer anorganischen Base ausgewählt aus Lithium-,Natrium-, Kalium-, Barium- oder Calciumhydroxid oder einer Mineralsäure ausgewählt aus Salz-, Schwefel- oder Bromwasserstoffsäure bei 0 °C bis 150 °C innerhalb von 1 bis 72 Stunden unter Rühren - gegebenenfalls gefolgt von anschließender Schutzgruppenentfernung in an sich üblicher Weise - zum Endprodukt der Formel I umsetzt und anschließend in an sich bekannter Weise isoliert.

3. Verfahren gemäß Anspruch 1 zur Herstellung von Verbindungen der allgemeinen Formel I mit R in der Bedeutung einer 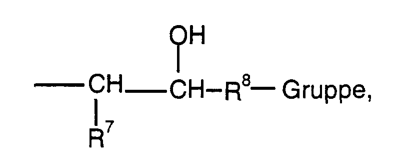 worin
R⁷ und R⁸ die in Anspruch 1 angegebene Bedeutung haben, **dadurch gekennzeichnet, daß** man Tetraazatricyclotridecan bzw. -pentadecan mit einem Edukt der allgemeinen Formel IV 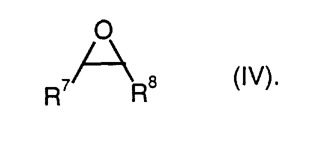
worin
R⁷ und R⁸ die oben angegebene Bedeutung haben, wobei gegebenenfalls vorhandene Hydroxy- und/oder Carboxylgruppen gegebenenfalls geschützt sind, mit oder ohne Lösungsmittel, wobei als Lösungsmittel aprotische Lösungsmittel ausgewählt aus Benzol, Toluol, Dichlormethan, Tetrahydrofuran, Dioxan, Acetonitril, Dimethylformamid, Dimethylacetamid, Dimethylsulfoxid, Hexan oder Ether verwendet werden, bei 0 °C bis 220 °C (wobei im Falle der höheren Umsetzungstemperatur das gegebenenfalls zum Lösen des zugesetzten Edukts der allgemeinen Formel IV verwendete Lösungsmittel vorher im Vakuum abdestilliert worden ist) oder in einem Autoklaven bei einem Überdruck von 1 bis 100 atm. innerhalb von 1 bis 48 Stunden umsetzt, anschließend das so erhaltene Reaktionsgemisch auf -20 °C bis 80 °C, abkühlt, mit einem Gemisch Wasser/organisches Lösungsmittel ausgewählt aus Methanol, Ethanol, iso-Propanol, Tetrahydrofuran oder Dioxan versetzt und 0,5 bis 12 Stunden bei -20 °C bis Raumtemperatur rührt, dann das so gebildete - gewünschtenfalls zu isolierende - eine Formylgruppe an einem Stickstoffatom tragende Zwischenprodukt durch Zugabe einer anorganischen Base ausgewählt aus Lithium-, Natrium-, Kalium-, Barium oder Calciumhydroxid oder einer Mineralsäure ausgewählt aus Salz-, Schwefel- oder Bromwasserstoffsäure bei 0 °C bis 150 °C innerhalb von 1 bis 72 Stunden unter Rühren - gegebenenfalls gefolgt von anschließender Schutzgruppenentfernung in an sich üblicher Weise - zum Endprodukt der Formel I umsetzt und anschließend in an sich bekannter Weise isoliert.

4. Verfahren gemäß Anspruch 1 zur Herstellung von Verbindungen der allgemeinen Formel I mit R in der Bedeutung eines 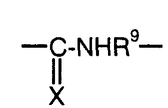 Restes,
worin
X und R⁹ die in Anspruch 1 angegebene Bedeutung haben, **dadurch gekennzeichnet, daß** man Tetraazatricyclotridecan bzw. -pentadecan mit einem Edukt der allgemeinen Formel V
R⁹-N=C=X (V),
worin
X und R⁹ die oben angegebene Bedeutung haben,
mit oder ohne Lösungsmittel wobei als Lösungsmittel aprotische Lösungsmittel ausgewählt aus Benzol , Toluol, Dichlormethan Tetrahydrofuran , Dioxan, Acetonitril , Dimethylformamid, Hexan oder Ether verwendet werden, bei 0 °C bis 180 °C (wobei im Falle der höheren Umsetzungstemperatur das gegebenenfalls zum Lösen des zugesetzten Edukts der allgemeinen Formel V verwendete Lösungsmittel vorher im Vakuum abdestilliert worden ist), innerhalb von 1 bis 48 Stunden umsetzt, anschließend das so erhaltene Reaktionsgemisch auf -20 °C bis 80 °C abkühlt, mit einem Gemisch Wasser/organisches Lösungsmittel ausgewählt aus Methanol, Ethanol, iso-Propanol, Tetrahydrofuran oder Dioxan versetzt und 0,5 bis 12 Stunden bei -20 °C bis Raumtemperatur rührt, dann das so gebildete - gewünschtenfalls zu isolierende - eine Formylgruppe an einem Stickstoffatom tragende Zwischenprodukt durch Zugabe einer anorganischen Base ausgewählt aus Lithium-, Natrium-, Kalium-, Barium- oder Calciumhydroxid oder einer Mineralsäure ausgewählt aus Salz-, Schwefel- oder Bromwasserstoffsäure bei 0 °C bis 150 °C innerhalb von 1 bis 72 Stunden unter Rühren zum Endprodukt der Formel I umsetzt und anschließend in an sich bekannter Weise isoliert.

5. Verfahren gemäß Anspruch 1 zur Herstellung von- Verbindungen der allgemeinen Formel I mit R in der Bedeutung eines -(CH₂)₂- NH-SO₂-R¹⁰-Restes,
worin
R¹⁰ die in Anspruch 1 angegebene Bedeutung hat, **dadurch gekennzeichnet, daß** man Tetraazatricyclotridecan bzw. -pentadecan mit einem Edukt der allgemeinen Formel VI 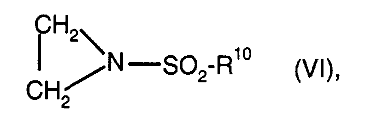
worin
R¹⁰ die oben angegebene Bedeutung hat mit Lösungsmittel, wobei als Lösungsmittel aprotische Lösungsmittel ausgewählt aus Benzol, Toluol, Dichlormethan, Tetrahydrofuran, Dioxan, Acetonitril, Dimethylformamid, Hexan oder Ether verwendet werden, bei 0 °C bis 180 °C (wobei im Falle der höheren Umsetzungstemperatur das verwendete Lösungsmittel vorher im Vakuum abdestilliert worden ist), innerhalb von 1 bis 48 Stunden umsetzt, anschließend das so erhaltene Reaktionsgemisch auf -20 °C bis 80 °C abkühlt, mit einem Gemisch Wasser/organisches Lösungsmittel ausgewählt aus Methanol, Ethanol, iso-Propanol, Tetrahydrofuran oder Dioxan versetzt und 0,5 bis 12 Stunden bei -20 °C bis Raumtemperatur rührt, dann das so gebildete - gewünschtenfalls zu isolierende - eine Formylgruppe an einem Stickstoffatom tragende Zwischenprodukt durch Zugabe einer anorganischen Base ausgewählt aus Lithium-, Natrium-, Kalium-, Barium- oder Calciumhydroxid oder einer Mineralsäure ausgewählt aus Salz-, Schwefel- oder Bromwasserstoffsäure bei 0 °C bis 150 °C innerhalb von 1 bis 72 Stunden unter Rühren zum Endprodukt der Formel I umsetzt und anschließend in an sich bekannter Weise isoliert.

6. Verfahren gemäß Anspruch 1 zur Herstellung von Verbindungen der allgemeinen Formel I mit R in der Bedeutung eines über eine Bis(β-hydroxy)-alkylenkette 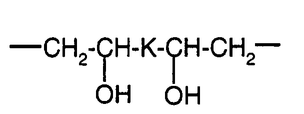 gebundenen zweiten 1,4,7,10-Tetraazacyclododecan- oder -tetradecan-Moleküls
worin
K die in Anspruch 1 angegebene Bedeutung hat, **dadurch gekennzeichnet, daß** man Tetraazatricyclotridecan bzw. -pentadecan mit einem Edukt der allgemeinen Formel VII 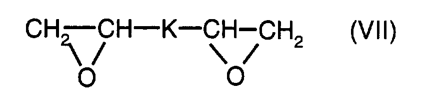
worin
K die oben angegebene Bedeutung hat, wobei gegebenenfalls vorhandene Hydroxygruppen gegebenenfalls geschützt sind, mit oder ohne Lösungsmittel, wobei als Lösungsmittel aprotische Lösungsmittel ausgewählt aus Benzol, Toluol, Dichlormethan, Tetrahydrofuran, Dioxan, Acetonitril, Dimethylformamid, Hexan oder Ether verwendet werden, bei 0 °C bis 220 °C, (wobei im Falle der höheren Umsetzungstemperatur das gegebenenfalls zum Lösen des zugesetzten Edukts der allgemeinen Formel VII verwendete Lösungsmittel vorher im Vakuum abdestilliert worden ist) oder in einem Autoklaven bei einem Überdruck von 1 bis 100 atm. innerhalb von 1 bis 48 Stunden umsetzt, anschließend das so erhaltene Reaktionsgemisch auf -20 °C bis 80 °C abkühlt, mit einem Gemisch Wasser/organisches Lösungsmittel, ausgewählt aus Methanol, Ethanol, iso-Propanol, Tetrahydrofuran oder Dioxan versetzt und 0,5 bis 12 Stunden bei -20 °C bis Raumtemperatur rührt, dann das so gebildete gewünschtenfalls zu isolierende - eine Formylgruppe an einem Stickstoffatom tragende Zwischenprodukt durch Zugabe einer anorganischen Base ausgewählt aus Lithium-. Natrium-, Kalium-, Barium oder Calciumhydroxid oder einer Mineralsäure ausgewählt aus Salz-, Schwefel- oder Bromwasserstoffsäure bei 0 °C bis 150 °C innerhalb von 1 bis 72 Stunden unter Rühren - gegebenenfalls gefolgt von anschließender Schutzgruppenentfernung in an sich üblicher Weise - zum Endprodukt der Formel I umsetzt und anschließend in an sich bekannter Weise, isoliert.

7. Verfahren nach Anspruch 1, **dadurch gekennzeichnet, daß** man die Reaktion ausgehend von 1,4,7,10-Tetraazacyclododecan oder 1,4,8,11-Tetraazacyclotetradecan ohne Isolierung der Zwischenprodukte zu den Verbindungen der allgemeinen Formel I durchführt.

8. Verfahren nach einem der Ansprüche 1 bis 7, **dadurch gekennzeichnet, daß** die hergestellten mono-N-Tetraazacyclododecan- und -tetradecan-Derivaten der allgemeinen Formel I weiter zu Metallkomplexen für die Diagnostik und Therapie verarbeitet werden.

## Claims

1. A process for preparing mono-N-substituted tetraazacyclododecane and -tetradecane derivatives of the formula I 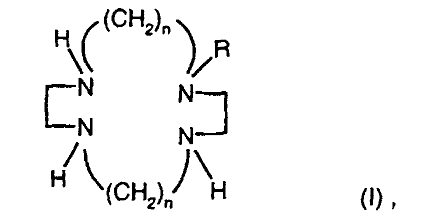 in which
n is the number 2 or 3,
R is a group -CR²R³ -CHR¹-A, -CHR⁷⁻CH (OH) -R⁸, -C(X)NHR⁹, -(CH₂)₂-NHSO₂R¹⁰ or is a second tetraazacyclododecane or -tetradecane molecule which is attached via -CH₂-CH(OH)-K-CH(OH)-CH₂-
in which
R¹ is a hydrogen atom, a straight-chain or cyclic C₁-C₆-alkyl, a phenyl or benzyl group - where the phenyl group may in each case be substituted by 1 or 2 chlorine, bromine, nitro, C₁-C₇-alkoxy or aralkoxy or CO₂R⁴ radicals, where R⁴ is a hydrogen atom, a C₁-C₆-alkyl, phenyl or benzyl group,
R² and R³ independently of one another are each R¹ or a CO₂R⁴ group,
A is a CN, CO₂R⁴ or 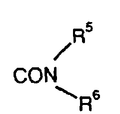 radical,
in which
R⁵ and R⁶ independently of one another are each a hydrogen atom, a saturated or unsaturated straight-chain, branched or cyclic hydrocarbon radical having up to 16 carbon atoms which is optionally substituted by 1 to 5 hydroxyl groups or by 1 or 2 CO₂R⁴ radicals and is optionally interrupted by 1 to 8 oxygen atoms or 1 to 3 phenylene or phenyleneoxy groups, are phenyl or benzyl radicals which are optionally substituted by 1 to 3 hydroxyl or C₁-C₆-alkoxy groups, or R⁵ and R⁶ together with the nitrogen atom are a saturated or unsaturated 5- or 6-membered ring which optionally contains a further nitrogen, oxygen or sulphur atom or a carbonyl group and which is optionally substituted by 1 to 3 C₁-C₆-alkyI radicals which are optionally substituted by 1 to 3 hydroxyl radicals, where any hydroxyl and/or carboxyl groups present are optionally protected,
R⁷ and R⁸ independently of one another are each a hydrogen atom, a C₁-C₂₀-alkyl radical which is optionally interrupted by 1 to 10 oxygen atoms, a phenylene, phenyleneoxy or phenylenedioxy group and which is optionally substituted by 1 to 3 C₁-C₆-alkyl, 1 to 3 trifluoromethyl, 1 to 7 hydroxyl, 1 to 3 C₁-C₇-alkoxy or -aralkoxy, 1 or 2 CO₂R⁴ and/or 1 or 2 phenoxy or phenyl groups, which are optionally substituted by 1 or 2 chlorine, bromine, nitro or C₁-C₆-alkoxy radicals, where any hydroxyl radicals present are optionally present in protected form,
X is an oxygen or sulphur atom and
R⁹ is a phenyl, 1- or 2-naphthyl or straight-chain or cyclic C₁-C₆-alkyl group,
R¹⁰ is a C₁-C₆-alkyl, -CF₃ or a phenyl group which is optionally substituted by a C₁-C₆-alkyl, chlorine, bromine or nitro radical, and
K is a C₀-C₁₆-alkylene chain which is optionally interrupted by 1 to 6 oxygen atoms, 1 or 2 phenylene, phenyleneoxy or phenylenedioxy groups and is optionally substituted by 1 to 6 hydroxyl, hydroxyalkyl or by 1 to 6 C₁-C₇-alkoxy, benzyloxy or aralkoxy groups, where carboxyl and hydroxyl groups are optionally present in protected form, **characterized in that** the compounds of the formula II 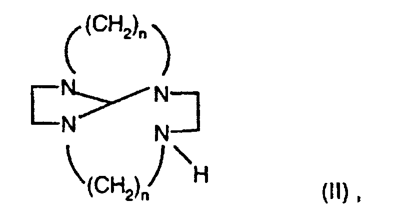
obtainable from 1,4,7,10-tetraazacyclododecane or 1,5,8,11-tetraazacyclotetradecane are reacted with an appropriate starting material of the formula 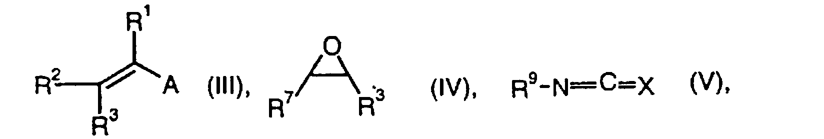 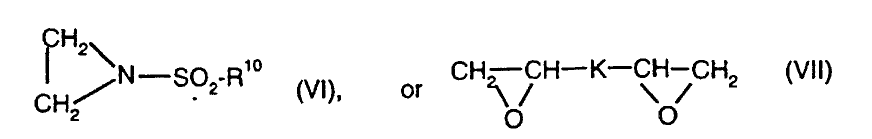 in which
R¹-R¹⁰, A, X and K are as defined above,
in the presence or absence of a solvent at from 0 to 220°C for from 1 to 48 hours, if appropriate at a pressure of up to 100 atm, the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring - if appropriate followed by subsequent removal of protective groups in a customary manner known per se - into the end product of the formula I and then isolated in a manner known per se.

2. Process according to Claim 1 for preparing compounds of the formula I
where
R is a 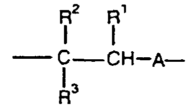 group,
in which
R¹, R², R³ and A are as defined in Claim 1,
**characterized in that** tetraazatricyclotridecane or -pentadecane is reacted with a starting material of the formula III 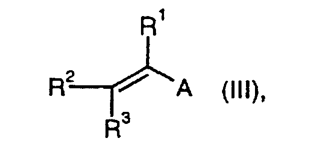 in which
R¹, R², R³ and A are as defined above, where any hydroxyl and/or carboxyl groups present are optionally protected, in the presence or absence of a solvent, suitable solvents being aprotic solvents selected from the group consisting of benzene, toluene, dichloromethane, tetrahydrofuran, dioxane, acetonitrile, dimethylformamide, hexane and ether, at from 0°C to 210°C (where, in the case of the higher reaction temperature, the solvent which may have been used for dissolving the added starting material of the formula III is distilled off under reduced pressure beforehand) for from 12 to 48 hours, the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring - if appropriate followed by subsequent removal of protective groups in a customary manner known per se - into the end product of the formula I and then isolated in a manner known per se.

3. Process according to Claim 1 for preparing compounds of the formula I where R is a 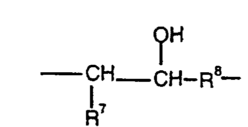 group,
in which
R⁷and R⁸ are as defined in Claim 1, **characterized in that** tetraazatricyclotridecane or -pentadecane is reacted with a starting material of the formula IV 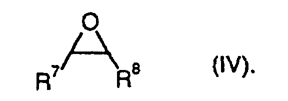
in which
R⁷ and R⁸ are as defined above, where any hydroxyl and/or carboxyl groups present are optionally protected, in the presence or absence of a solvent, suitable solvents being aprotic solvents selected from the group consisting of benzene, toluene, dichloromethane, tetrahydrofuran, dioxane, acetonitrile, dimethylformamide, dimethylacetamide, dimethyl sulphoxide, hexane and ether, at from 0°C to 220°C (where, in the case of the higher reaction temperature, the solvent which may have been used for dissolving the added starting material of the formula IV is distilled off under reduced pressure beforehand) or in an autoclave at a gauge pressure of from 1 to 100 atm for from 1 to 48 hours, the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring - if appropriate followed by subsequent removal of protective groups in a customary manner known per se - into the end product of the formula I and then isolated in a manner known per se.

4. Process according to Claim 1 for preparing compounds of the formula I where R is a 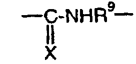 radical,
in which
X and R⁹ are as defined in Claim 1, **characterized in that** tetraazatricyclotridecane or -pentadecane is reacted with a starting material of the formula V
R⁹-N=C=X (V),
in which
X and R⁹ are as defined above, in the presence or absence of a solvent, suitable solvents being aprotic solvents selected from the group consisting of benzene, toluene, dichloromethane, tetrahydrofuran, dioxane, acetonitrile, dimethylformamide, hexane and ether, at from 0°C to 180°C (where, in the case of the higher reaction temperature, the solvent which may have been used for dissolving the added starting material of the formula V is distilled off under reduced pressure beforehand) for from 1 to 48 hours, the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring into the end product of the formula I and then isolated in a manner known per se.

5. Process according to Claim 1 for preparing compounds of the formula I where R is a -(CH₂)₂-NH-SO₂-R¹⁰- radical,
in which
R¹⁰ is as defined in Claim 1, **characterized in that** tetraazatricyclotridecane or -pentadecane is reacted with a starting material of the formula VI 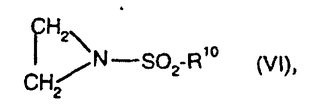
in which
R¹⁰ is as defined above, is in the presence of a solvent, suitable solvents being aprotic solvents selected from the group consisting of benzene, toluene, dichloromethane, tetrahydrofuran, dioxane, acetonitrile, dimethylformamide, hexane and ether, at from 0°C to 180°C (where in the case of the higher reaction temperature, the solvent used is distilled off under reduced pressure beforehand) for from 1 to 48 hours the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring into the end product of the formula I and then isolated in a manner known per se.

6. Process according to Claim 1 for preparing compounds of the formula I where R is a second 1,4,7,10-tetraazacyclododecane or -tetradecane molecule attached via a bis(β-hydroxy)alkylene chain 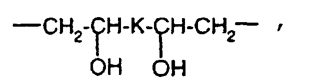 in which
K is as defined in Claim 1, **characterized in that** tetraazatricyclotridecane or -pentadecane is reacted with a starting material of the formula VII 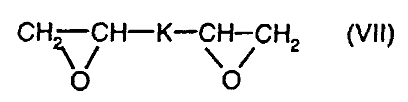
in which
K is as defined above, where any hydroxyl groups present are optionally protected, in the presence or absence of a solvent, suitable solvents being aprotic solvents selected from the group consisting of benzene, toluene, dichloromethane, tetrahydrofuran, dioxane, acetonitrile, dimethylformamide, hexane and ether, at from 0°C to 220°C (where, in the case of the higher reaction temperature, the solvent which may have been used for dissolving the added starting material of the formula VII is distilled off under reduced pressure beforehand) or in an autoclave at a gauge pressure of from 1 to 100 atm for from 1 to 48 hours, the resulting reaction mixture is then, after cooling to from -20°C to 80°C, treated with a mixture of water/organic solvent selected from the group consisting of methanol, ethanol, isopropanol, tetrahydrofuran and dioxane, and stirred at from -20°C to room temperature for from 0.5 to 12 hours, the resulting intermediates, which carry a formyl group on a nitrogen atom (and which may be isolated, if desired) are, by addition of an inorganic base selected from lithium hydroxide, sodium hydroxide, potassium hydroxide, barium hydroxide and calcium hydroxide or a mineral acid selected from hydrochloric, sulphuric or hydrobromic acid, converted at from 0 to 150°C over a period of from 1 to 72 hours with stirring - if appropriate followed by subsequent removal of protective groups in a customary manner known per se - into the end product of the formula I and then isolated in a manner known per se.

7. Process according to Claim 1, **characterized in that** the reaction is carried out starting with 1,4,7,10-tetraazacyclododecane or 1,4,8,11-tetraazacyclotetradecane, without isolation of the intermediates, to give the compounds of the formula I.

8. Process according to any of Claims 1 to 7, **characterized in that** the mono-N-tetraazacyclododecane and -tetradecane derivatives of the formula I are processed further to metal complexes for diagnosis and therapy.

## Revendications

1. Procédé pour la préparation de dérivés de tétraazacyclododécane et de tétraazacyclotétradécane mono-N-substitués de formule générale I : 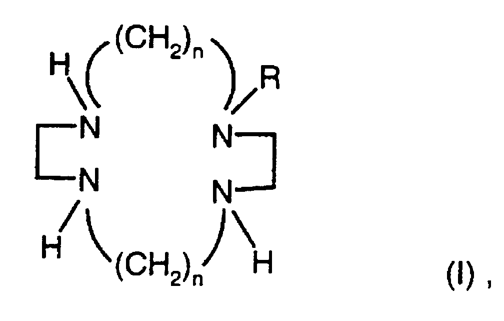 dans laquelle :
n est égal à 2 ou 3,
R désigne un groupement -CR²R³ -CHR¹-A, -CHR⁷-CH(OH)-R⁸, -C(X)NHR⁹, -(CH₂)₂-NHSO₂R¹⁰ ou une deuxième molécule de tétraazacyclododécane ou de tétraazacyclotétradécane liée par le biais de -CH₂-CH(OH)-K-CH(OH)-CH₂-,
dans laquelle :
R¹ désigne un atome d'hydrogène, un groupement alkyle en C₁-C₆ à chaîne droite ou cyclique, phényle ou benzyle, dans lequel le groupement phényle peut être substitué respectivement par 1 à 2 radicaux chloro, bromo, nitro, alcoxy ou aralcoxy en C₁-C₇ ou CO₂R⁴, R⁴ désignant un atome d'hydrogène, un groupement alkyle en C₁-C₆, phényle ou benzyle,
R² et R³ désignent, indépendamment l'un de l'autre, respectivement, R¹ ou un groupement CO₂R⁴,
A désigne un radical CN, CO₂R⁴ ou CONR⁵R⁶,
dans laquelle :
R⁵ et R⁶ désignent, indépendamment l'un de l'autre, respectivement un atome d'hydrogène, un radical hydrocarbure avec jusqu'à 16 atomes de carbone saturé, insaturé, à chaîne droite ou ramifiée ou cyclique substitué par 1 à 5 groupements hydroxy ou par 1 à 2 radicaux CO₂R⁴, le cas échéant interrompu par 1 à 8 atomes d'oxygène ou par 1 à 3 groupements phénylène ou phénylèneoxy, le cas échéant des radicaux phényle ou benzyle substitués par 1 à 3 groupements hydroxy ou alcoxy en C₁-C₆, ou R⁵ et R⁶, conjointement avec l'atome d'azote, désignent un noyau à cinq ou six chaînons, saturé ou insaturé, contenant le cas échéant un autre atome d'azote, d'oxygène, de soufre ou un groupement carbonyle, ledit noyau étant le cas échéant substitué par 1 à 3 radicaux alkyle en C₁-C₆ substitués le cas échéant par 1 à 3 radicaux hydroxy, les groupements hydroxy et/ou carboxy le cas échéant présents étant protégés le cas échéant,
R⁷ et R⁸ désignent, indépendamment l'un de l'autre, respectivement un atome d'hydrogène, un radical alkyle en C₁-C₂₀ interrompu le cas échéant par 1 à 10 atomes d'oxygène, un groupement phénylène, phénylèneoxy ou phénylènedioxy, qui est substitué le cas échéant par 1 à 3 groupements alkyle en C₁-C₆, 1 à 3 groupements trifluorométhyle, 1 à 7 groupements hydroxy, 1 à 3 groupements alcoxy ou aralcoxy en C₁-C₇, 1 à 2 groupements CO₂R⁴ et/ou 1 à 2 groupements phénoxy ou phényle substitués le cas échéant par 1 à 2 radicaux chloro, bromo, nitro ou alcoxy en C₁-C₆, les radicaux hydroxy le cas échéant présents se présentant le cas échéant sous une forme protégée,
X est un atome d'oxygène ou de soufre, et
R⁹ est un groupement phényle, 1 ou 2-naphtyle ou alkyle en C₁-C₆ à chaîne droite ou cyclique,
R₁₀ désigne un groupement alkyle en C₁-C₆, -CF₃- ou un groupement phényle le cas échéant substitué par un radical alkyle en C₁-C₆, chloro, bromo ou nitro, et
K désigne une chaîne alkylène en C₀-C₁₆ le cas échéant interrompue par 1 à 6 atomes d'oxygène, 1 à 2 groupements phénylène, phénylèneoxy ou phénylènedioxy, le cas échéant substituée par 1 à 6 groupements hydroxy, hydroxyalkyle ou 1 à 6 groupements alcoxy en C₁-C₇, benzyloxy, ou aralcoxy, les groupements carboxyle et hydroxy étant le cas échéant présents sous une forme protégée, **caractérisé en ce qu'**on fait réagir les composés de formule générale II obtenus à partir de 1,4,7,10-tétraazacyclododécane ou selon le cas de 1,5,8,11-tétraaza-cyclotétradécane : 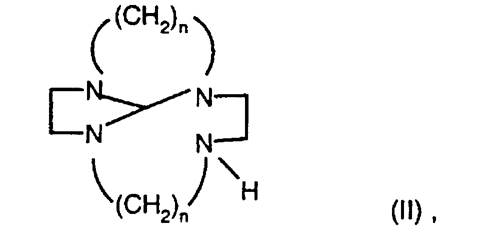
avec un éduit correspondant de formules générales : 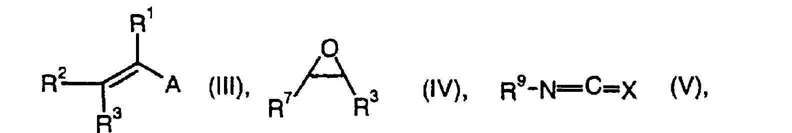 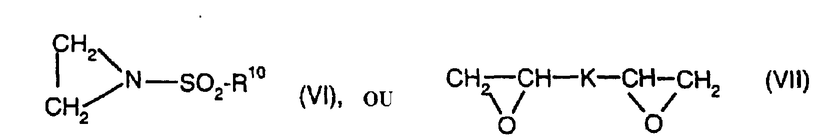 dans lesquelles :
R¹-R¹⁰, A, X et K ont la signification indiquée ci-dessus,
avec ou sans solvant à 0 à 220°C, en l'espace de 1 à 48 heures, le cas échéant sous une pression allant jusqu'à 100 atmosphères, ensuite le mélange réactionnel ainsi obtenu est mélangé, après refroidissement à -20°C jusqu'à 80°C, avec un mélange d'eau et de solvant organique choisi parmi le méthanol, l'éthanol, l'isopropanol, le tétrahydrofuranne ou le dioxanne et on agite le tout pendant 0,5 à 12 heures à une température allant de -20°C jusqu'à la température ambiante, puis on convertit les produits intermédiaires ainsi formés - à isoler si on le souhaite - portant un groupement formyle sur un atome d'azote par addition d'une base inorganique, choisie parmi les hydroxydes de lithium, de sodium, de potassium, de baryum ou de calcium ou d'un acide minéral choisi parmi l'acide chlorhydrique, l'acide sulfurique ou l'acide bromhydrique à 0 à 150°C, en l'espace de 1 à 72 heures, sous agitation - le tout étant suivi le cas échéant d'une élimination consécutive des groupes de protection de manière usuelle en soi - en produit final de formule I qui est ensuite isolé de manière connue en soi.

2. Procédé selon la revendication 1 pour la préparation de composés de formule générale I où R signifie dans un groupe de formule : 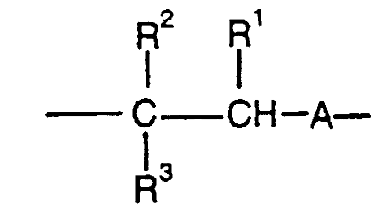 dans laquelle :
R¹, R², R³ et A ont la signification indiquée dans la revendication 1, **caractérisé en ce que** l'on fait réagir du tétraazatricyclotridécane ou selon le cas du tétraazatricyclopentadécane avec un éduit de formule générale III : 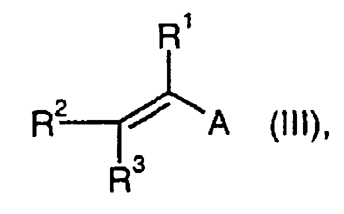
dans laquelle :
R¹, R², R³ et A ont la signification indiquée ci-dessus, dans laquelle les groupements hydroxy et/ou carboxyle le cas échéant présents sont le cas échéant protégés, avec ou sans solvant, dans laquelle on utilise comme solvant des solvants aprotiques choisis parmi le benzène, le toluène, le dichlorométhane, le tétrahydrofuranne, le dioxanne, l'acétonitrile, le diméthylformamide, l'hexane ou l'éther, à 0°C jusqu'à 210°C (où, dans le cas où la température réactionnelle est plus élevée, le solvant utilisé le cas échéant pour dissoudre l'éduit ajouté de formule générale III a été préalablement séparé par distillation sous vide), en l'espace de 12 à 48 heures, ensuite le mélange réactionnel ainsi obtenu est refroidi à -20 jusqu'à 80°C, mélangé avec un mélange d'eau et de solvant organique choisi parmi le méthanol, l'éthanol, l'isopropanol, le tétrahydrofuranne ou le dioxanne, et agité pendant 0,5 à 12 heures à -20°C jusqu'à la température ambiante, puis le produit intermédiaire ainsi formé - à isoler si on le souhaite - portant un groupement formyle sur un atome d'azote est converti, par addition d'une base inorganique choisie parmi les hydroxydes de lithium, de sodium, de potassium, de baryum ou de calcium ou à partir d'un acide minéral choisi parmi les acides chlorhydrique, sulfurique et bromhydrique à 0 jusqu'à 150°C, en l'espace de 1 à 72 heures sous agitation - le tout étant le cas échéant suivi d'une élimination des groupes de protection de manière usuelle en soi - en produit final de formule I et ensuite isolé de manière connue en soi.

3. Procédé selon la revendication 1 pour la préparation de composés de formule générale I où R signifie dans un groupe de formule : 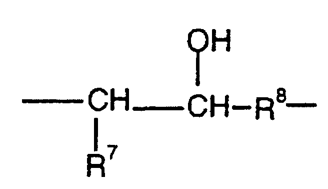 dans laquelle :
R⁷ et R⁸ ont la signification indiquée dans la revendication 1, **caractérisé en ce qu'**on fait réagir du tétraazatricyclotridécane ou selon le cas du tétraazatricylcopentadécane avec un éduit de formule générale IV : 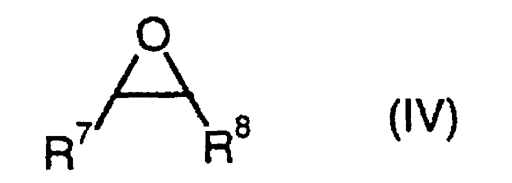
dans laquelle :
R⁷ et R⁸ ont la signification indiquée ci-dessus,
dans laquelle des groupements hydroxy et/ou carboxyle le cas échéant présents sont le cas échéant protégés, avec ou sans solvant, dans laquelle on utilise comme solvant des solvants aprotiques choisis parmi le benzène, le toluène, le dichlorométhane, le tétrahydrofuranne, le dioxanne, l'acétonitrile, le diméthylformamide, le diméthylacétamide, le sulfoxyde de diméthyle, l'hexane ou l'éther, à 0° jusqu'à 220°C (où, dans le cas où la température réactionnelle est plus élevée, le solvant utilisé le cas échéant pour dissoudre l'éduit ajouté de formule générale IV a été séparé préalablement par distillation sous vide) ou dans un autoclave à une surpression de 1 à 100 atmosphères dans l'intervalle de 1 à 48 heures, ensuite le mélange réactionnel ainsi obtenu est refroidi à -20 jusqu'à 80°C, mélangé à un mélange d'eau et de solvant organique choisi parmi le méthanol, l'éthanol, l'isopropanol, le tétrahydrofuranne ou le dioxanne et agité pendant 0,5 à 12 heures à une température de -20°C jusqu'à la température ambiante, puis le produit intermédiaire ainsi formé - à isoler si on le souhaite - portant un groupement formyle sur un atome d'azote est converti par addition d'une base inorganique choisie parmi les hydroxydes de lithium, de sodium, de potassium, de baryum ou de calcium ou d'un acide minéral choisi parmi l'acide chlorhydrique, l'acide sulfurique ou l'acide bromhydrique à 0°C jusqu'à 150°C en l'espace de 1 à 72 heures sous agitation - le tout étant le cas échéant suivi d'une élimination consécutive des groupes de protection de manière usuelle en soi - en produit final de formule I et est ensuite isolé de manière connue en soi.

4. Procédé selon la revendication 1 pour la préparation de composés de formule générale I où R signifie un radical de formule : 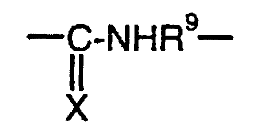 dans laquelle :
X et R⁹ ont la signification indiquée dans la revendication 1, **caractérisé en ce qu'**on fait réagir du tétraazatricyclotridécane ou selon le cas du tétraazatricyclopentadécane avec un éduit de formule générale V :
R⁹-N=C=X (V)
dans laquelle :
X et R⁹ ont la signification indiquée ci-dessus, avec ou sans solvant, dans laquelle on utilise comme solvant des solvants aprotiques choisis parmi le benzène, le toluène, le dichlorométhane, le tétrahydrofuranne, le dioxanne, l'acétonitrile, le diméthylformamide, l'hexane ou l'éther, à 0°C jusqu'à 180°C (où, dans le cas où la température réactionnelle est plus élevée, le solvant utilisé le cas échéant pour dissoudre l'éduit ajouté de formule générale V a été séparé préalablement par distillation sous vide), dans l'intervalle de 1 à 48 heures, ensuite le mélange réactionnel ainsi obtenu est refroidi à -20 jusqu'à 80°C, mélangé à un mélange d'eau et de solvant organique choisi parmi le méthanol, l'éthanol, l'isopropanol, le tétrahydrofuranne ou le dioxanne et agité pendant 0,5 à 12 heures à une température de -20°C jusqu'à la température ambiante, puis le produit intermédiaire ainsi formé - à isoler si on le souhaite - portant un groupement formyle sur un atome d'azote est converti par addition d'une base inorganique choisie parmi les hydroxydes de lithium, de sodium, de potassium, de baryum ou de calcium ou d'un acide minéral choisi parmi l'acide chlorhydrique, l'acide sulfurique ou l'acide bromhydrique à 0°C jusqu'à 150°C en l'espace de 1 à 72 heures sous agitation en produit final de formule I et ensuite isolé de manière connue en soi.

5. Procédé selon la revendication 1 pour la préparation de composés de formule générale I où R signifie un radical de formule :
-(CH₂)₂-NH-SO₂-R¹⁰-
dans laquelle :
R¹⁰ a la signification indiquée dans la revendication 1, **caractérisé en ce qu'**on fait réagir du tétraazatricyclotridécane ou selon le cas du tétraazatricyclopentadécane avec un éduit de formule générale VI : 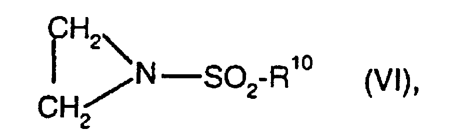
dans laquelle :
R¹⁰ a la signification indiquée ci-dessus, avec un solvant, dans laquelle on utilise comme solvant des solvants aprotiques choisis parmi le benzène, le toluène, le dichlorométhane, le tétrahydrofuranne, le dioxanne, l'acétonitrile, le diméthylformamide, l'hexane ou l'éther, à 0°C jusqu'à 180°C (où, dans le cas où la température réactionnelle est plus élevée, le solvant utilisé a été séparé préalablement par distillation sous vide), dans l'intervalle de 1 à 48 heures, ensuite le mélange réactionnel ainsi obtenu est refroidi à -20 jusqu'à 80°C, mélangé à un mélange d'eau et de solvant organique choisi parmi le méthanol, l'éthanol, l'isopropanol, le tétrahydrofuranne ou le dioxanne et agité pendant 0,5 à 12 heures à une température de -20°C jusqu'à la température ambiante, puis le produit intermédiaire ainsi formé - à isoler si on le souhaite - portant un groupement formyle sur un atome d'azote est converti par addition d'une base inorganique choisie parmi les hydroxydes de lithium, de sodium, de potassium, de baryum ou de calcium ou d'un acide minéral choisi parmi l'acide chlorhydrique, l'acide sulfurique ou l'acide bromhydrique, à 0°C jusqu'à 150°C, en l'espace de 1 à 72 heures sous agitation, en produit final de formule I et ensuite isolé de manière connue en soi.

6. Procédé selon la revendication 1 pour la préparation de composés de formule générale I où R signifie une seconde molécule de 1,4,7,10-tétraazacyclododécane ou de tétraazacyclotétradécane liée via une chaîne de bis(β-hydroxy)alkylène de formule : 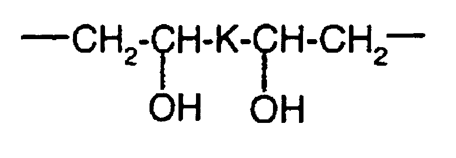 dans laquelle :
K a la signification indiquée dans la revendication 1, **caractérisé en ce qu'**on fait réagir du tétraazatricyclotridécane ou selon le cas du tétraazatricyclopentadécane avec un éduit de formule générale VII : 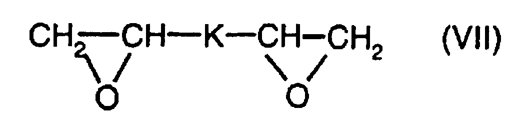
dans laquelle :
K a la signification indiquée ci-dessus, dans laquelle les groupements hydroxy le cas échéant présents sont le cas échéant protégés, avec ou sans solvant, dans laquelle on utilise comme solvant des solvants aprotiques choisis parmi le benzène, le toluène, le dichlorométhane, le tétrahydrofuranne, le dioxanne, l'acétonitrile, le diméthylformamide, l'hexane ou l'éther, à 0°C jusqu'à 220°C (où, dans le cas où la température réactionnelle est plus élevée, le solvant utilisé le cas échéant pour dissoudre l'éduit ajouté de formule générale VII a été séparé préalablement par distillation sous vide), ou dans un autoclave sous une surpression de 1 à 100 atmosphères dans l'intervalle de 1 à 48 heures, ensuite le mélange réactionnel ainsi obtenu est refroidi à -20°C jusqu'à 80°C, mélangé à un mélange d'eau et de solvant organique choisi parmi le méthanol, l'éthanol, l'isopropanol, le tétrahydrofuranne ou le dioxanne et agité pendant 0,5 à 12 heures à une température de -20°C jusqu'à la température ambiante, puis le produit intermédiaire ainsi formé - à isoler si on le souhaite - portant un groupement formyle sur un atome d'azote est converti par addition d'une base inorganique choisie parmi les hydroxydes de lithium, de sodium, de potassium, de baryum ou de calcium ou d'un acide minéral choisi parmi l'acide chlorhydrique, l'acide sulfurique ou l'acide bromhydrique à 0°C jusqu'à 150°C en l'espace de 1 à 72 heures sous agitation - le tout étant le cas échéant suivi d'une élimination consécutive des groupes de protection de manière usuelle en soi - en produit final de formule I et ensuite isolé de manière connue en soi.

7. Procédé selon la revendication 1, **caractérisé en ce que** l'on effectue la réaction à partir de 1,4,7,10-tétraazacyclododécane ou de 1,4,8,11-tétraazacyclotétradécane sans isolement des produits intermédiaires pour obtenir les composés de formule générale I.

8. Procédé selon l'une quelconque des revendications 1 à 7, **caractérisé en ce que** les dérivés de mono-N-tétraazacyclododécane et de mono-N-tétraazacyclotétradécane préparés de formule générale I sont encore convertis en complexes métalliques pour le diagnostic et la thérapie.
